# EUROPEAN PATENT SPECIFICATION

(11) **EP 2 967 801 B1**
(45) Date of publication and mention of the grant of the patent: **08.07.2020**
(21) Application number: 14773377.8
(22) Date of filing: 13.03.2014
(51) Int. Cl.: A61L 24/00, A61L 24/02, A61K 35/32, A61K 38/05, A61K 38/08, A61K 38/12, A61K 38/17, A61K 31/05, A61L 24/10

(54) **INJECTABLE BIODEGRADABLE BONE MATRIX FOR MULTIPLE MYELOMA LESION AUGMENTATION AND OSTEOPOROSIS**
INJIZIERBARE BIOLOGISCH ABBAUBARE KNOCHENMATRIX ZUR LINDERUNG MEHRERER MYELOMLÄSIONEN UND VON OSTEOPOROSE
MATRICE OSSEUSE BIODÉGRADABLE INJECTABLE POUR TRAITER PAR AUGMENTATION DES LÉSIONS DE MYÉLOME MULTIPLE ET L'OSTÉOPOROSE

(30) Priority: 14.03.2013 US 201361783100 P
(43) Date of publication of application: 20.01.2016
(73) Proprietor: The University of Toledo, Toledo, OH 43606 (US)
(72) Inventor: AGARWAL, Anand, K., Toledo, OH 43606 (US); GOEL, Vijay, K., Toledo, OH 43606 (US); KIM, Dong-Shik, Toledo, OH 43606 (US); BHADURI, Sarit, B., Toledo, OH 43606 (US); LIN, Boren, Toledo, OH 43606 (US)
(74) Representative: Comoglio, Elena
(86) International application number: PCT/US2014/026099
(87) International publication number: WO 2014/160232

(56) References cited:
- WO-A1-2011/116085
- WO-A1-2011/116085
- WO-A1-2011/139136
- WO-A2-2011/137292
- US-A1- 2011 130 468
- KAZUHIRO AOKI ET AL: "Peptide-based delivery to bone", ADVANCED DRUG DELIVERY REVIEWS, vol. 64, no. 12, 1 September 2012 (2012-09-01), pages 1220-1238, XP055307966, AMSTERDAM, NL ISSN: 0169-409X, DOI: 10.1016/j.addr.2012.05.017
- CHARLOTTE A.E. HAUSER ET AL: "Designer Self-Assembling Peptide Materials for Diverse Applications", MACROMOLECULAR SYMPOSIA., vol. 295, no. 1, 1 September 2010 (2010-09-01), pages 30-48, XP055284383, DE ISSN: 1022-1360, DOI: 10.1002/masy.200900171
- SAHITHI K ET AL: "Polymeric composites containing carbon nanotubes for bone tissue engineering", INTERNATIONAL JOURNAL OF BIOLOGICAL MACROMOLECULES, ELSEVIER BV, NL, vol. 46, no. 3, 1 April 2010 (2010-04-01), pages 281-283, XP026937518, ISSN: 0141-8130, DOI: 10.1016/J.IJBIOMAC.2010.01.006 [retrieved on 2010-03-03]
- HAUSER ET AL.: 'Designer Self-Assembling Peptide Materials for Diverse Applications.' MACROMOLECULAR SYMPOSIA vol. 295, no. 1, 2010, pages 30 - 48, XP055284383
- SAHITHI ET AL.: 'Polymeric composites containing carbon nanotubes for bone tissue engineering' INT J BIOL MACROMOL. vol. 46, no. 3, 2010, pages 281 - 283, XP026937518

## Description

### BACKGROUND OF THE INVENTION

Bone metabolism can be affected by various medical conditions. These conditions include primary diseases such as metastatic tumors in the skeleton, which increase bone resorbing substances. Bone lesions and fractures can also be side effects of therapies given to treat a primary condition. For example, hormone treatments for breast or prostate cancer may induce osteoporosis.

Multiple myeloma is the second most common blood cancer, and causes osteoporotic destruction. Osteoporotic destruction, a major complication in multiple myeloma patients, has significant influence on quality of life. The underlying mechanism is the continuous breakdown of bone tissue by bone-resorbing (osteoclast) cells in the abnormal bone marrow microenvironment created by malignant cells. At present, treatment is made by applying conventional PMMA cement, which merely stabilizes the fractured vertebrae but does not support or induce bone regeneration and may cause injury in surrounding tissue due to its inert and exothermic properties.

WO 2011/139136 A1 discloses a bone filler which is a calcium phosphate based cement comprising hydroxyl-functionalized multiwalled carbon nanotubes and cells or growth factors.

In addition, current medications treating imbalanced bone remodeling processes given via the patient's circulatory system have resulted in significant complications. Thus, there is a need in the art for additional and improved treatments of multiple myeloma, as well as other cancer-induced bone diseases.

### SUMMARY OF THE INVENTION

Provided herein is a bone filler composition comprising an alkaline earth phosphate-based cement, nanotubular structures and at least one of a therapeutic agent or cells, as defined in appended claim 1. The composition of the invention is a paste that sets into a hardened mass, and the hardened mass is capable of releasing the therapeutic agent or cells.

In certain embodiments, the nanotubular structures comprise peptide nanotubes: In particular embodiments, the peptide nanotubes are formed from Glyₙ-Aspₙ peptides, wherein each n is independently from 1 to 10 [SEQ ID NO: 3]. In particular embodiments, the peptide nanotubes comprise the peptide Gly-Gly-Gly-Gly-Gly-Gly-Asp-Asp [SEQ ID NO: 2] (also referred to herein as Gly6-Asp2). In particular embodiments, the peptide nanotubes comprise a compound selected from the group consisting of: N,N'-bis(glycyl-glycine)-hexane-1,6-dicarboxyamide; N,N'-Bis (glycylglycylglycine)octadecane-1,18-dicarboxyamide; and N,N'-bis(glycylglycine)decane-1,10- dicarboxyamide. In particular embodiments, the peptide nanotubes comprise ionic complementary peptides. In some embodiments, the ionic complementary peptides comprise diphenylalanine.

According to the invention, the nanotubular structures comprise carboxyl-functionalized multi-walled carbon nanotubes.

In certain embodiments, the nanotubular structures comprise a blend of peptide nanotubes and carbon nanotubes.

In certain embodiments, the therapeutic agent comprises a compound having the structural formula of Formula I: 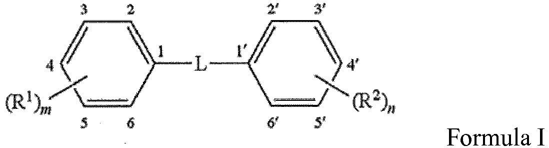 wherein L is a linkage between the two phenyl rings selected from a -C=C- acetylene linkage, a-C≡C-ethylene linkage, or a -C-C- ethane linkage; R1 and R2 are each independently substituents at any available position of the phenyl rings; m and n are each independently 0, 1, 2, 3, 4, or 5, and at least one of m or n is ≧1; and R1 and R2 are each independently selected from the group consisting of: -OH;
a halogen; a haloalkyl group with one C atom substituted with from 1 to 3 halogen atoms; a C₁-C₆ alkyl; a C₂-C₆ alkenyl; a C₂C₆ alkynyl group; and -OR³, wherein R³ is a C₁-C₆ alkyl, a C₂-C₆ alkenyl, or a C₂-C₆ alkynyl group; wherein at least one occurrence of R¹ or R² is - OH, provided that when Lis -C=C-, the compound of Formula I is not resveratrol. In particular embodiments, the therapeutic agent is selected from the group consisting of: 4,4'-dihydroxytolan (KST-201); 4-hydroxy-4'-trifluoromethyltolan or 4'- hydroxy-4-trifluoromethyltolan (KST-213); 3,4',5-trihydroxytolan or 3',4,5'-trihydroxytolan (KST-301); and 3,3',5,5'-tetrahydroxytolan (KST-401). In particular embodiments, at least one occurrence of R¹ or R² is -OH, provided that (i) when L is -C=C-, the compound of Formula I is a trans-stilbene and is not resveratrol; (ii) when L is -C=C-, the compound is not KST-201, KST-213, KST-301, or KST-401; and (iii) when L is -C-C-, the compound is not a 1-(2,6-dichloro-4-hydroxyphenyl)-2-phenylethane.

In certain embodiments, the therapeutic agent comprises an antagonist of a signal transduction responsible for osteoclast differentiation or activation. In certain embodiments, the therapeutic agent is selected from the group consisting of bisphosphonates and RANKL inhibitors. In certain embodiments, the therapeutic agent comprises one or more of: Alendronate, Zolendronate, Pamidronate, Denosumab, Ibandronate, zolendronic acid, and Bortezomib. In certain embodiments, the therapeutic agent comprises a peptide having the sequence YCWSQYLCY [**SEQ ID NO: 1**]. In certain embodiments, the therapeutic agent is selected from the group consisting of: bisphosphonates, estrogens, hormones, proteasome inhibitors, osteoclast-promoting cytokine antagonists, and phytochemicals. In certain embodiments, the therapeutic agent is selected from the group consisting of: alendronate, alendronate, ibandronate, zoledrbnic acid, raloxifene, denosumab, teriparatide, pamidronate disodium, bortezomib, cadilzomib, cyclophosphamide, doxorubicin hydrochloride liposome, lenalidomide, plerixafor, pomalidomide, Salinosporamide A, MG132, infliximab, adalimumab, certolizumab pegol, golimumab, etanercept, xanthine derivatives, hallucinogens, isoflavones, lignans, flavones, flavanols, flavonols, flavanones, catechins, epigallocatechin gallate (EGCG), stilbenes, vitamin B, vitamin D, and vitamin K.

In certain embodiments, the cells comprise mesenchymal stem cells or osteoblasts derived from mesenchymal stem cells. In particular embodiments, the mesenchymal stem cells or osteoblasts are encapsulated in alginate beads. In particular embodiments, the alginate beads comprise carboxy methyl cellulose.

According to the invention, the alkaline earth phosphate-based cement consists essentially of monetite, CaHPO₄.

In certain embodiments, the nanotubular structures are present at a concentration ranging from about 0.1 wt% to about 5.0 wt% of the composition. In certain embodiments, the nanotubular structures are present at a concentration ranging from about 0.5 wt% to about 2.0 wt% of the composition. In certain embodiments, the nanotubular structures are present at a concentration of about 1 wt% of the composition. In certain embodiments, the composition is an injectable paste.

Further provided herein is a method of preparing a bone filler material complex as defined in appended claim 11. The method of the invention comprises the steps of preparing an alkaline earth phosphate-based bone cement powder consisting essentially of monetite, mixing the powder with nanotubes and pre-dissolved therapeutic agents to form a paste, wherein the nanotubes comprise carboxyl-functionalized multi-walled carbon nanotubes, and adding encapsulated cells to the paste to form a complex. In certain embodiments, the cells are encapsulated in alginate beads. In celtain embodiments, the nanotubes comprise peptide nanotubes.

Further provided herein is a method of treating osteoporosis, the method comprising the steps of: collecting mesenchymal stem cells from a patient having osteoporosis through a cell differentiation procedure; preparing osteoblasts from the mesenchymal stem cells; incorporating the osteoblasts into a bone filler composition comprising an alkaline earth phosphate-based cement and nanotubular structures; and delivering the bone filler composition to the patient to treat osteoporosis. This method is not part of the invention.

Further provided herein is a method of treating multiple myeloma-induced bone diseases and the cancer itself, the method comprising the steps of: incorporating an antagonist of signal transduction responsible for osteoclast differentiation and/or activation, or a therapeutic agent inhibiting myeloma tumor progression into a bone filler composition comprising an alkaline earth phosphate-based cement and nanotubular structures; and delivering the bone filler composition to a patient in need thereof. This method is not part of the invention.

Further provided herein is a method to treat a cancer-induced bone defect, the method comprising injecting a composition described herein into a bone defect of a subject in need thereof, and allowing the composition to set into a hardened mass. In certain embodiments, the method further comprises performing a bone augmentation procedure. This method is not part of the invention.

Further provided herein is a kit for preparing a bone filler composition comprising a first container housing an alkaline earth phosphate-based cement, a second container housing a source of nanotubes, and a third container housing a therapeutic agent or cells. In certain embodiments, the kit comprises a therapeutic agent and cells in separate containers. In certain embodiments, the kit further comprises alginate and a source of multivalent cations. In certain embodiments, the kit further comprises a syringe configured to inject a cement paste.

### BRIEF DESCRIPTION OF THE DRAWING

**FIG. 1**: Exemplary illustration of the preparation and delivery of a cement/nanotube/drug/osteoblast complex via a bone augmentation technique.
**FIG. 2**: Exemplary illustration of the inhibition of bone resorption and restoration of bone formation by a drug-loaded, osteoblast-incorporated cement composition.
**FIGS. 3A-3B**: Photographs of cement with nanotubes (**FIG. 3A**) and cement alone (**FIG. 3B**) extruded from a syringe and evaluated for handling properties. These photographs show the injectability of cement paste was retained when incorporated with carbon nanotubes.
**FIGS. 4A-4B**: Incorporation of carbon nanotubes improved the mechanical properties of calcium phosphate cement. **FIG. 4A** shows the compressive strength (MPa) of cement with varying concentrations of nanotubes. **FIG. 4B** shows the Young's modulus of cement with varying concentrations of nanotubes. These figures show the optimal mechanical strength was achieved with cement having 1 wt% nanotubes. (DCPA: monetite calcium phosphate cement alone; mCNTs: multi- walled carboxyl-functionalized carbon nanotube; untreated: multi-walled carbon nanotube without carboxyl group.)
**FIGS. 5A-5D**: Seven day culture of osteoblasts on calcium phosphate cement (**FIGS. 5A-5B**) and calcium phosphate cement/multi-walled carbon nanotube (**FIGS**. **5C-5D**).
**FIG. 6**: When loaded with anti-osteoclast WP9QY [**SEQ ID NO: 1**], a potential drug candidate for this treatment, wherein the cement immersed in preosteoclast cell culture can inhibit osteoclast activation. The data were obtained by counting TRAP positive cells, a marker for active osteoclasts, after stimulated by the cytokine TNFalpha.
**FIGS. 7A-7B**: SEM images ofnanotubes formed by a self-assembled surfactant-like peptide Gly-Gly-Gly-Gly-Gly-Gly-Asp-Asp [SEQ ID NO: 2], a member of Glyₙ-ASPₙ-These images depict the formation of nano-scale tubular structures.
**FIG. 8**: Drug release profile of the drug MG132 when loaded in a carbon nanotube (MWCNT)/cement composite. MG132 was added into the cement and left to release to the cell culture medium for a certain time period as indicated. This medium was used to culture indicator cells follow by critical staining to indicate live cells. A toxic burst release effect was showed when CPC was used as a drug carrier, but this burst release was attenuated when the carbon nanotubes were present.
**FIG. 9**: Improvement of drug release with carbon nanotubes present. MG132 was left to release in the culture medium for a certain time period as indicated after loaded on a cement. This medium was added to the cells pre-transfected with NF-kB promoter-containing luciferase reporters to measure inhibition of cytokine-mediated NF-kB activation. The results indicated a more sustained release of the drug when carbon nanotubes were added.

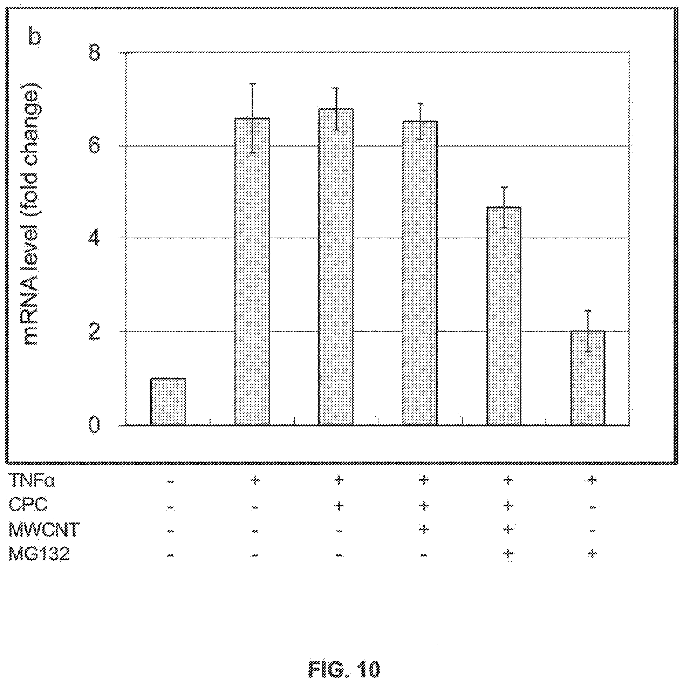

**FIG. 10**: MG132/MWCNT/CPC complex blocking osteoclast differentiation.
**FIG.11**: MTT assay used to measure cell viability when the stem cells were incubated with surfactant-like peptide Gly-Gly-Gly-Gly-Gly-Gly-Asp-Asp [SEQ ID NO: 2]-formed nanotubes for 1 and 3 days. No significant cytotoxicity was observed, indicating this material is safe to use in tissue.
**FIGS. 12A-12B****:** SEM images of the structure of diphenylalanine nanotubes (FIG. 12A) and when incorporated with CPC **(****FIG. 12B****).**
**FIGS. 13A-13D****:** Mesenchymal stem cells accumulated on diphenylalanine nanotubes in culture, indicating their biocompatibility.

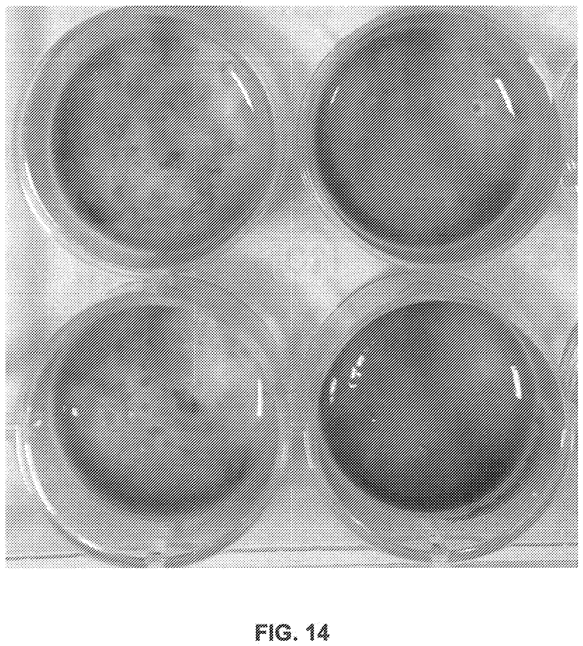

**FIG.14****:** Photograph showing indicator cells can attach and proliferate on a diphenylalanine nanotube/CPC composite in a 3 day culture period. Live cells were detected by MTT.
**FIG. 15**: Osteoblast differentiation of mesenchymal stem cells cultured in carboxyl methyl cellulose/magnesium ion/alginate beads by the BCIPINBT method measuring ALP activity.
Various aspects of this invention will become apparent to those skilled in the art from the following detailed description, when read in light of the accompanying drawings.

### DETAJLED DESCRIPTION OF THE INVENTION

### DEFINITIONS

The term "peptide" as used herein encompasses native peptides (either degradation products, synthetically synthesized peptides, or recombinant peptides) and peptidomimetics (typically, synthetically synthesized peptides), as .well as peptoids and semipeptoids, which are peptide analogs and may have, for example, modifications rendering the peptides more stable while in a body or more capable of penetrating into cells. Such modifications include, but are not limited to: N terminus modification, C telminus modification, or peptide bond modifications. Peptide bond modifications include, but are not limited to: CH₂NH, CH₂S, CH₂S=O, O=C-NH, CH₂O, CH₂-CH₂, S=C-NH, CH=CH, CF=CH, backbone modifications, and residue modifications. Methods for preparing peptidomimetic compounds are known in the art.

Peptide bonds (-CO-NH-) within the peptide may be substituted, for example, by: N- methylated bonds (-N(CH₃)-CO-); ester bonds (-C(R)H-C-O-O-C(R)-N-); ketomethylen bonds (-CO-CH₂-); α-aza bonds (-NH-N(R)-CO-), wherein R is any alkyl; carba bonds (-CH₂-NH-); hydroxyethylene bonds (-CH(OH)-CH₂-); thioamide bonds (-CS-NH-); olefinic double bonds (-CH=CH-); retro amide bonds (-NH-CO-); and peptide derivatives (-N(R)-CH₂-CO-), wherein R is the "normal" side chain, naturally presented on the carbon atom. These modifications can occur at any of the bonds along the peptide chain and even at several (2-3) at the same time.

Natural aromatic amino acids, Trp, Tyr, and Phe, may be substituted for synthetic non-natural acids such as Phenylglycine, TIC, naphthylalanine (Nal), ring-methylated derivatives of Phe, halogenated derivatives of Phe, o-methyl-Tyr, and βamino acids.

In addition to the above, the peptides described herein may also include one or more modified amino acids (e.g., thiolated amino acids or biotinylated amino acids), or one or more non-amino acid monomers (e.g. fatty acids, complex carbohydrates etc). Also contemplated are homodipeptides, such as aromatic homodipeptides in which each of the amino acids comprises an aromatic moiety. These include, but are not limited to: substituted or unsubstituted naphthalenyl and substituted or unsubstituted phenyl. The aromatic moiety can alternatively be a substituted or unsubstituted heteroaryl such as, for example: indole, thiophene, imidazole, oxazole, thiazole, pyrazole, pyridine, pyrimidine, quinoline, isoquinoline, quinazoline, quinoxaline, or purine. When substituted, the phenyl, naphthalenyl, or any other aromatic moiety, includes one or more substituents such as, but not limited to: alkyl, trihaloalkyl, alkenyl, alkynyl, cycloalkyl, aryl, heteroaryl, heteroalicyclic, halo, nitro, azo, hydroxy, alkoxy, thiohydroxy, thioalkoxy, cyano, and amine.

As used herein, the term "alkyl" refers to a saturated aliphatic hydrocarbon including straight chain and branched chain groups. Preferably, the alkyl group has from 1 to 20 carbon atoms. The alkyl group may be substituted or unsubstituted. When substituted, the substituent group can be, for example, trihaloalkyl, alkenyl, alkynyl, cycloalkyl, aryl, heteroaryl, heteroalicyclic, halo, nitro, azo, hydroxy, alkoxy, thiohydroxy, thioalkoxy, cyano, or amine.

As used herein, a "cycloalkyl" group refers to an all-carbon monocyclic or fused ring (i.e., rings which share an adjacent pair of carbon atoms) group wherein one or more of the rings does not have a completely conjugated pi-electron system. Examples, without limitation, of cycloalkyl groups include cyclopropane, cyclobutane, cyclopentane, cyclopentene, cyclohexane, cyclohexadiene, cycloheptane, cycloheptatriene, and adamantane. A cycloalkyl group may be substituted or unsubstituted. When substituted, the substituent group can be, for example, alkyl, trihaloalkyl, alkenyl, alkynyl, cycloalkyl, aryl, heteromyl, heteroalicyclic, halo, nitro, azo, hydroxy, alkoxy, thiohydroxy, thioalkoxy, cyano, or amine.

As used herein, an "alkenyl" group refers to an alkyl group which consists of at least two carbon atoms and at least one carbon-carbon double bond.

As used herein, an "alkynyl" group refers to an alkyl group which consists of at least two carbon atoms and at least one carbon-carbon triple bond.

As used herein, an "aryl" group refers to an all-carbon monocyclic or fused-ring polycyclic (i.e., rings which share adjacent pairs of carbon atoms) groups having a completely conjugated pi-electron system. Examples, without limitation, of aryl groups include phenyl, naphthalenyl, and anthracenyl. The aryl group may be substituted or unsubstituted. When substituted, the substituent group can be, for exatnple, alkyl, trihaloalkyl, alkenyl, alkynyl, cycloalkyl, atyl, heteroaryl, heteroalicyclic, halo, nitro, azo, hydroxy, alkoxy, thiohydroxy, thioalkoxy, cyano, or amine.

As used herein, a "heteroaryl" group refers to a monocyclic or fused ring (i.e., rings which share an adjacent pair of atoms) group having in the ring(s) one or more atoms, such as, for example, nitrogen, oxygen, or sulfur and, in addition, having a completely conjugated pi-electron system. Exatnples, without limitation, ofheteroaryl groups include pynole, furane, thiophene, imidazole, oxazole, thiazole, pyrazole, pyridine, pyrimidine, quinoline, isoquinoline, and purine. The heteroaryl group may be substituted or unsubstituted. When substituted, the substituent group can be, for example, alkyl, trihaloalkyl, alkenyl, alkynyl, cycloalkyl, aryl, heteroaryl, heteroalicyclic, halo, nitro, azo, hydroxy, alkoxy, thiohydroxy, thioalkoxy, cyano, or amine.

As used herein, a "heteroalicyclic" group refers to a monocyclic or fused ring group having in the ring(s) one or more atoms such as nitrogen, oxygen, and sulfur. The rings may also have one or more double bonds. However, the rings do not have a completely conjugated pi-electron system. The heteroalicyclic may be substituted or unsubstituted. When substituted, the substituted group can be, for example, lone pair electrons, alkyl, trihaloalkyl, alkenyl, alkynyl, cycloalkyl, aryl, heteroaryl, heteroalicyclic, halo, nitro, azo, hydroxy, alkoxy, thiohydroxy, thioalkoxy, cyano, or amine.
Representative examples include piperidine, piperazine, tetrahydro furane, tetrahydropyrane, morpholino, and the like.

A "hydroxy" group refers to an -OH group.

An "azide" group refers to a -N=N=N group.

An "alkoxy" group refers to both an -O-alkyl and an -O-cycloalkyl group, as defined herein.

An "aryloxy" group refers to both an -O-aryl and an -O-heteroaryl group, as defined herein.

A "thiohydroxy," group refers to an -SH group.

A "thioalkoxy" group refers to both an -S-alkyl group, and an -S-cycloalkyl group, as defined herein.

A "thioaryloxy" group refers to both an -S-aryl and an -S-heteroaryl group, as defined herein.

A "halo" group refers to fluorine, chlorine, bromine, or iodine.

A "trihaloalkyl" group refers to an alkyl substituted by three halo groups, as defined herein. A representative example is trihalomethyl.

An "amino" group refers to an -NR'R" group where R' and R" are each independently hydrogen, alkyl, cycloalkyl, or aryl.

A "nitro" group refers to an -NO₂ group.

A "cyano" group refers to a -C≡N group.

The acronym "CPC" as used herein refers to a calcium phosphate-based cement composition. The acronym "MPC" as used herein refers to a magnesium phosphate-based cement composition.

### GENERAL DESCRIPTION

Conventional treatments of cancer-induced bone defects, such as applying PMMA bone cement at vertebral fractures, do not support or induce growth of new bone. While curing, PMMA releases heat, causing injury in surrounding tissue. In addition, the inert property of this material may obstruct nutrient supply to the intervertebral discs. PMMA merely stabilizes the fractured vertebrae and, thus, is not the best interest of the patient in long term. In the case of multiple myeloma bone marrow, because excessive osteoclast-activating cytokines are continuously produced, the bone is subject to osteolytic breakdown. At the same time, some of these cytokines can also inhibit bone formation mediated by osteoblasts. Therefore, the overall effect of multiple myeloma is to sustain an environment favorable to bone resorption. An ideal approach to treating such defects should thus target both ends of the bone remodeling process by introducing anti-osteolysis therapies to suppress osteoporosis and supplying active osteoblasts to restore bone formation.

The invention relates to a biodegradable alkaline earth phosphate-based composition containing nano-tubular structures, the composition being capable of carrying, and sustainably releasing, drugs and/or cells such as osteoblasts, the composition being as defined in appended claim 1. The composition can be used for many medical applications, such as, but not limited to, spine surgery for the treatment of osteoporosis lesions or fractures, and for the treatment of cancer-induced bone defects like multiple myeloma lesions. The composition comprises an injectable bone filler material as defined in claim 1 that is biocompatible for bone-forming (osteoblast) cells, and can carry therapeutic agents for localized administration to inhibit bone resorption. This alleviates many current issues with bone augmentation methods that are used for treating bone defects such as multiple myeloma-induced fractures.

The bone filler material comprises an alkaline earth phosphate-based cement consisting essentially of monetite mixed with nanotubes, or nano-scale cylinders. This complex can be prepared as an injectable paste for use in the operation of bone augmentation, along with therapeutic agents such as antagonists of osteoclasts, and alginate-encapsulated osteoblasts. A calcium phosphate-based cement, consisting essentially of monetite, is an ideal substitute for PMMA because it is biodegradable, osteoconductive, and minimally endothermic when solidifying. When incorporated with nanotubes, the cement retains suitable mechanical and handling properties comparable to conventional materials used in bone augmentation operations. This combination serves as a scaffold when injected and cured in the cavity of damaged bone, allowing bone-forming osteoblasts (when present in the composition) to attach and grow while stabilizing the fractured sites. After injection, the cement cures at the site of fractures, providing load bearing in order to stabilize the damaged vertebrae. The nanotubes reinforce the cement scaffold, improve its handling properties, and increase its drug delivery capabilities. The composition can include any combination of one or more therapeutic agents such as osteolysis agents, and/or cells mediating osteogenesis, for delivery from the hardened cement mass.

As a whole, the composition exhibits a syngergistic effect to protect bone tissue from multiple myeloma or other disease-induced damage. The composition is injectable, and serves as a load- bearing scaffold for bone augmentation operations. Tests have shown the composition to have
mechanical and handling properties comparable to PMMA, and to be osteoconductive, biodegradable, and less exothermic than PMMA. The composition is useful for many applications, such as, but not limited to, in conjunction with a bone augmentation surgery to treat cancer-induced bone disease.

### Cement Component

A alkaline earth phosphate-based cement consisting essentially of monetite, CaHPO₄OO cements, is used in the bone filler material of the invention.

The alkaline earth phosphate-based cements used in the bone filler material of the invention include cement compositions that are irradiated by microwaves prior to being allowed to set into a hardened mass. Alternatively, the paste could be allowed to set without irradiation.

The alkaline earth phosphate-based cement used in the invention serving as the scaffold plays an important role in load bearing for the bone augmentation operation. Its mechanical and handling properties have been proven comparable to PMMA. Other tests have shown that this material is minimally endothermic, osteoconductive, and biodegradable.
In addition, its drug-canying capability has also been confirmed.

### Nanotubular Structures

Nanotubular structures can be incorporated into the cement composition to improve its strength, toughness, ductility, fatigue resistance, and macroporosity. Furthermore, incorporating nanotubes improves the burst release issue of loaded drugs on calcium phosphate cement. Suitable nanotubular structures for use the cement composition include, but are not limited to, various types of carbon nanotubes, various types of peptide nanotubes, and combinations thereof.

Carbon nanotubes are cylindrical structures with unique mechanical and chemical properties. The carbon nanotubes can be used to form scaffolds for bone cell proliferation, and can be functionalized to serve as carriers for therapeutic agents. Types of carbon nanotubes suitable for use in the cement composition of the present disclosure include, but are not limited to, carboxyl- or amino-functionalized single-walled (SWNT), multi-walled (MWNT), and ultra-short carbon nanotubes. SWNTs are long- wrapped graphene sheets, and MWNTs are generally a collection of concentric SWNTs having different diameters. SWNTs typically have a length-to-diameter ratio of about 1,000, and as such are considered nearly one-dimensional. These nanotubes consist of two separate regions with different physical and chemical properties. A first region is the side wall of the tube and a second region is the end cap of the tube. The end cap structure is similar to a smaller fullerene, such as C₆₀.

Self-assembled peptides, a group of peptides that form nanostructures spontaneously, exhibit biochemical activities that are useful in biomedical applications. Because of their hollow cores, internal cavities, high affinity to biomolecules, encapsulating effect, diffusible property through the tube, controllable degradation rate, and well-ordered alignment, peptide nanotubes can be used for drug delivery, regenerative medicine, scaffold fabrication in tissue engineering, antimicrobial coatings, and medical diagnosis. Additionally, peptide nanotubes are easily chemically modified. The presence of amino acids in the nanotubes provides a bioactive site to interact with the living system. Suitable peptide nanotubes include, but are not limited to: surfactant like molecules; dipeptides; bolaamphiphiles (ionic complementary peptides); stacked D-,L- peptide subunits; and combinations thereof.

One non-limiting example of a peptide nanotube is formed from a peptide containing hydrophobic tails of glycines and hydrophilic heads of aspartic acids. These surfactant-like peptides can self-assemble to form peptide nanotubes because of the tail-head structure. Glycine is the simplest amino acid, but is an important building block of the most proteins. It has only one hydrogen, so it is very stable and will not convert to other amino acids. Aspartic acid has carboxyl groups and is charged, so the interaction of the polar head and the glycine tail results in self-assembly of a strong peptide nanotube.
The combination of these two amino acids is achieved via a relatively simple synthesis procedure. It provides excellent biocompatibility, as well as mechanical and chemical stability. In addition, the numbers of glycine and aspartic acid residues can be readily manipulated to control the degradation rate of the peptide nanotube. Without wishing to be bound by theory, it is believed that cylindrical subunits are formed from surfactant peptides that self-assemble into bilayers, where hydrophilic head groups remain exposed to the aqueous medium. The tubular arrays undergo self-assembly through non-covalent interactions that are widely found in surfactant and micelle structures and formation processes. In certain embodiments, these peptide nanotubes are composed of7-8 residues and are approximately 2 nm in length when fully extended, making them dimensionally similar to phospholipids found in cell membranes.

Other non-limiting examples of peptide nanotubes are those formed by self-assembly of dipeptides and bolaamphiphiles. Dipeptides contain either two amino acids joined by a single peptide bond, or one amino acid with two peptide bonds and other functional groups such as phenyl groups in diphenylalanine. Bolaamphiphiles have two hydrophilic peptides on both ends of a hyd ophobic alkyl linker and modified amino acids, which form nanotubes by ionic complementarity.

Other peptide-based nanotubular structures can be used in the composition. For example, peptide-based nanotubes can be made through stacking of cyclic D-, L-peptide subunits. These peptides self-assemble through hydrogen bonding interactions into nanotubules, which in tum self-assemble into ordered parallel arrays of nanotubes. As another example, peptide-based N,N'- bis(glycylglycylglycin)hexane-1,6-dicarboxyamide molecules assemble into tubular structures.

Some non-limiting examples of compounds that can be formed into peptide nanotubes include, but are not limited to: N,N'-bis(glycyl-glycine)-hexane-1,6-dicarboxyamide; N,N'-Bis (glycylglycylglycine)octadecane-1,18-dicarboxyamide; N,N'-bis(glycylglycine)decane-1,10- dicarboxyamide; and combinations thereof. In certain embodiments, the peptide nanotubes are generated by surfactant-like molecules such as, but not limited to, synthetic Glyₙ-Aspₙ peptides, where each n is independently any integer. In particular embodiments, n is from 1 to 10. In certain embodiments, the peptide nanotubes comprise ionic complementary peptides such as, but are not limited to, diphenylalanine. The skilled practitioner will recognize that the above-recited compounds are given for exemplary purposes, and many other types of peptide nanotubes are possible and encompassed within the present disclosure.

Proper blending of carbon nanotubes and/or peptide nanotubes can enhance the mechanical properties of the cement and increase its capability to carry biomolecules or drugs. Therefore, certain embodiments of the composition described herein include a blend of carbon nanotubes and peptide nanotubes. The proper amount of nanotubes can be added directly when the cement paste is in preparation prior to injection. The concentration of the nanotubes can range from about 0.1 wt% to about 5 wt% of the composition. In certain embodiments, the concentration of nanotubes ranges from about 0.5 wt% to about 2 wt%. In certain embodiments, the concentration of nanotubes is about 1 wt%.

### Therapeutic Agents

The composition of the present disclosure can carry a wide variety of therapeutic agents. Suitable therapeutic agents include, but are not limited to: antiresorptive drugs for the treatment of imbalanced bone metabolism such as bisphosphonates (alendronate, alendronate, ibandronate, zoledronic acid, or the like), estrogens, hormones, raloxifene, and denosumab; anabolic drugs for the treatment of imbalanced bone metabolism such as teriparatide and the like; drugs for the treatment of multiple myeloma and other plasma cell neoplasms such as pamidronate disodium, bortezomib, carfilzomib, cyclophosphamide, doxorubicin hydrochloride liposome, lenalidomide, plerixafor, pomalidomide, and the like; proteasome inhibitors such as Salinosporamide A, MG132, and the like; osteoclast-promoting cytokine antagonists such as infliximab, adalimumab, certolizumab pegol, golimumab, etanercept, xanthine derivatives, hallucinogens, and the small peptide WP9QY **[SEQ ID NO: 1];** phytochemicals such as isoflavones, lignans, flavones, flavanols, flavonols, flavanones, catechins, epigallocatechin gallate (EGCG), and stilbenes; or vitamin B, D, K, and the like. Additionally, the therapeutic agent can be a compound of Formula **1:** 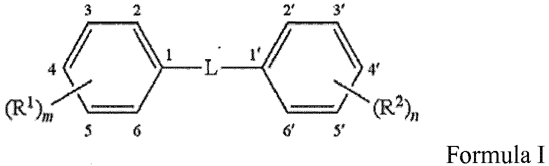 wherein L represents a linkage between the two phenyl rings selected from a -C≡C-acetylene linkage, a -C=C-ethylene linkage or a -C-C- ethane linkage; R¹ and R² are, independently, substituents at any available position of the phenyl rings; m and n are, independently, 0, 1, 2, 3, 4, or 5, representing the number of R¹ and R² substituents of the rings, respectively, and at least one of m or n is ≧1; wherein R¹ and/or R² is: -OH, a halogen, a haloalkyl group with one C atom substituted with from 1 to 3 halogen atoms, a C₁-C₆ alkyl, C₂-C₆ alkenyl, or C₂C₆ alkynyl group, -OR³, wherein R³ is a C₁-C₆ alkyl, C₂C₆ alkenyl, or C₂C₆ alkynyl group; and at least one occurrence of R¹ or R² is -OH, with the proviso that when L is -C=C-, the compound of Formula I is not resveratrol (otherwise known as 3,5,4'- trihydroxy-trans-stilbene).

The compounds of Formula I include KST201, which has the structural formula of Formula II:

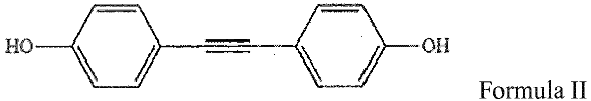

The compounds of Formula I further include KST-213, which has the structural formula of Formula III:

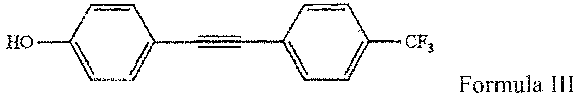

The compounds of FormulaI further include KST-401, which has the structural formula of Formula IV:

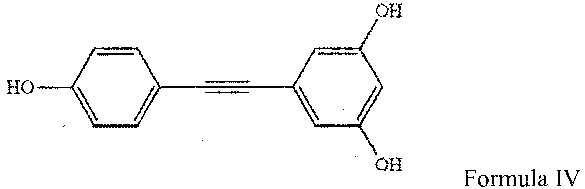

The compounds of FormulaI further include KST-401, which has the structural formula of Formula V:

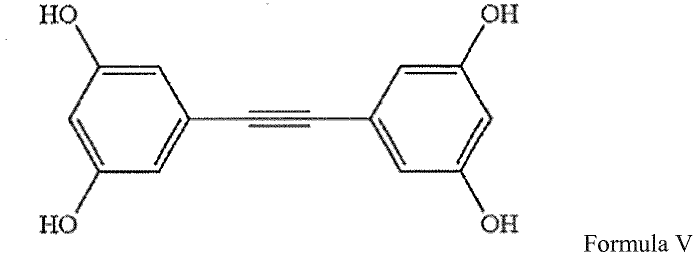

Suitable therapeutic agents further include compounds of Formula I: 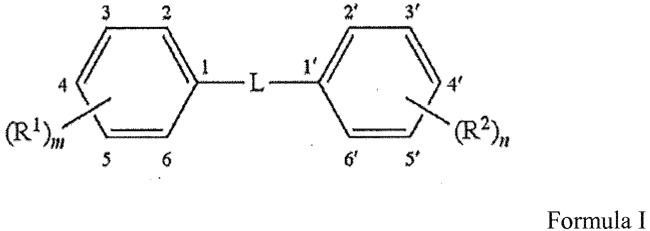 wherein: L represents a linkage between the two phenyl rings selected from a -C≡C-acetylene linkage, a -C=C- ethylene linkage or a -C-C- ethane linkage; R¹ and R² are, independently, substituents at any available position of the phenyl rings; m and n are, independently, 0, 1, 2, 3, 4, or 5, representing the number of R¹ and R² substituents of the rings, respectively, and at least one of m or n is ≧1; and R¹ and/or R² is: -OH, a halogen, a haloalkyl group with one C atom substituted with from 1 to 3 halogen atoms, a C₁-C₆ alkyl, C₂-C₆ alkenyl, or C₂C₆ alkynyl group, -OR³, wherein R³ is a C₁-C₆ alkyl, C₂-C₆ alkenyl, or C₂-C₆ alkynyl group; wherein at least one occurrence of R¹ or R² is -OH, with the proviso that (i) when L is -C=C-, the compound of Formula I is a trans-stilbene and is not resveratrol (3,5,4'-trihydroxy-trans-stilbene); (ii) when L is -C≡C-, the compound is not KST-201, KST-213, KST-301, or KST-401, and (iii) when L is -C-C-, the compound is not a 1-(2,6-dichloro-4-hydroxyphenyl)-2-phenylethane.

By way of a non-limiting example, the cement can carry inhibitors against osteolysis-promoting signal pathways. These small molecules can be concentrated and delivered locally at the site of fractures to directly interact with their targets, which are present in, or secreted by, myeloma cells, osteoclasts, and other cells in the bone marrow, and are responsible for the imbalance bone remodeling. In embodiments wherein the cells are autologous mesenchymal stem cell-derived osteoblasts, the anti- osteolysis drugs can accelerate the bone-forming process by working synergistically with the osteoblasts to enhance bone regeneration.

Another non-limiting example of a therapeutic agent in the composition is one designed to treat osteoporosis. To suppress osteoporosis by blocking bone resorption, an antagonist of the signal transduction responsible for osteoclast differentiation and activation, which can be a small compound, peptide, or protein, can be loaded in the alkaline earth phosphate-based cement/nanotube composite. The antagonist can then be delivered locally to the.site of treatment instead of via circulation, which is highly advantageous. Suitable anti-osteoclastic drugs that can be used for this purpose include, but are not limited to: bisphosphonates, such as Alendronate, Zolendronate, Ibandronate, Pamidronate, and zolendronic acid; RANKL inhibitors, such as Denosumab; and proteasome inhibitors, such as Bortezomib.

In the examples described herein, WP9QY [**SEQ ID NO: 1**] and MG132 were used as model therapeutic agents in the composition. WP9QY [**SEQ ID NO: 1**] was developed based on structural analysis of the ligand binding site of TNF receptor 1 and RANK to inhibit osteoclast-activating signal pathways mediated by these two receptors. MG132 is a proteasome inhibitor used to interrupt degradation of the inhibitor of NF-kB to prevent NF-kB activation and its activity of inducing gene. expression responsible for osteoclastogenesis and osteolytic activity. As non-limiting examples, the therapeutic agents incorporated into the composition can also target myeloma cells to induce cell death or inhibit tumor progress, again being administered directly to the location of the treatment instead of via circulation.

Because the bone filler material is not limited to use for the treatment of cancer-induced bone disease, many other therapeutic agents can be incorporated into the composition, depending on the desired therapeutic effect. Suitable therapeutic agents for use in the bone filler material include, but are not limited to: anti-osteoclastic agents, peptides, proteins, nucleic acids, antibiotics, antimicrobials, antifungals, antiseptics, antivirals, antiparasitics, antiprotozoals, wound healing agents, local anaesthetics, mucolytics, anti-allergies, antioxidants, vitamins, steroidal and non-steroidal antiinflammatory agents, antihistamines, chemotherapeutic agents, tumor growth inhibitory agents, cytostatics, and combinations thereof. Any of the therapeutic agents can be added directly to the cement paste at the desired dosage.

### Cells

To further restore the bone-forming capacity for damage repair, cells such as mesenchymal stem cells or osteoblasts can be incorporated into the bone filler material and implanted along with the alkaline earth phosphate-based cement/nanotube composition (which may also include one or more therapeutic agents). Osteoblast cells can be prepared *in vitro* from mesenchymal stem cells collected from the patient by a cell differentiation procedure with proper growth factors. The sources of mesenchymal stem cells used to prepare osteoblasts can be autologous or allogeneic. These cells are multipotent stromal cells that can differentiate into osteoblasts, chondrocytes, and adipocytes when exposed to the proper growth factors. By way of non-limiting example, the ficoll purification method can be used to isolate mononuclear cells from bone marrow harvested from the donor or the patient followed by cell culturing to obtain mesenchymal cell colonies. These stem cells can differentiate into osteoblasts when cultured with BMP-2.

Alginate beads are useful to protect the cells while they are delivered during a bone augmentation process along with the cement. Alginate's biocompatibility and simple gelation with divalent cations, such as Ca²⁺ or Mg²+, make it suitable for cell immobilization and encapsulation.
Gelation of alginate is achieved by divalent cation crosslinking for encapsulation of the cells to be carried. When mixing with the cement paste, the cell/alginate beads retain their structure, providing a three- dimensional matrix for cell proliferation inside the hardened cement. This cell encapsulating system consisting of alginate can be prepared by adding divalent cations, any of the nanotubes described herein, and carboxy methyl cellulose, which functions as a reservoir for osteoblast differentiation-promoting positive ions or other compounds. Thus, any of the compositions described herein may contain osteoblasts encapsulated in alginate beads. As a non-limiting example of a method to encapsulate cells in alginate for delivery, osteoblasts can be resuspended in 2% sodium alginic acid salt solution.
Alginate/cell beads can be made by adding the suspension drop wise into 2% calcium chloride. These can then be injected along with the cement paste.

Although osteoblasts are described for explanatory purposes, other cells can be incorporated into the composition, depending on the desired treatment or a therapeutic effect. Other suitable types of cells include, but are not limited to, chondrocytes, adipocytes, stromal cells, undifferentiated mesenchymal stem cells, and cells transfected with therapeutic agents and/or nucleic acids.

### Additives

In addition to the cement component, nanotubes, therapeutic agent, and cells, various additives may be included in the bone filler composition to adjust its properties. Examples of suitable additives include, but are not limited to: proteins, osteoinductive and/or osteoconductive materials, X-ray opacifying agents such as strontium phosphate or strontium oxide, supporting or strengthening filler materials, crystal growth adjusters, viscosity modifiers, pore-forming agents, antibiotics, antiseptics, growth factors, chemotherapeutic agents, bone resorption inhibitors, color change agents, immersing liquids, carboxylates, carboxylic acids, a-hydroxyl acids, metallic ions, or mixtures thereof. Other suitable additives include substances that adjust the setting time of the cement (such as pyrophosphates or sulfates), increase injectability or cohesion (such as hydrophobic polymers like collagen), alter swelling time, or introduce macroporosity (such as porogens).

### Methods of Use

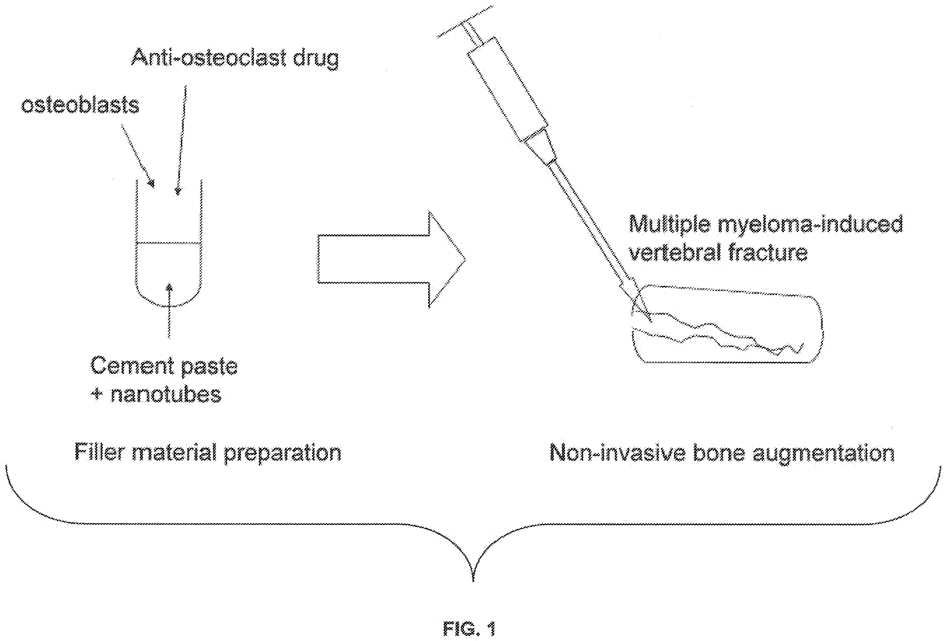

**FIG. 1** illustrates a non-limiting example of the preparation and use of a bone filler composition. As seen in **FIG. 1**, in one embodiment, an anti-osteoclast drug is added along with osteoblasts to a mixture of an alkaline earth phosphate-based cement and nanotubes. This mixture is then injected into a multiple myeloma-induced vertebral fracture during a non-invasive bone augmentation. Once injected, the cement hardens to provide load bearing in order to stabilize the vertebra. Bone- forming osteoblasts attach and grow while stabilizing the fractured site. The nanotubes reinforce the cured scaffold and allow for increased drug delivery from the cement mixture.

The composition of the present disclosure provides a promising solution for cancer-induced bone diseases, such as, but not limited to, multiple myeloma-induced bone diseases. A crucial drawback of current medications targeting this imbalanced bone remodeling process involves side effects due to administration of the therapeutic agent via circulatory system. Combined with non-invasive bone augmentation techniques, such as kyphoplasty or vertebroplasty, the implant of the present disclosure functions as a vehicle that can deliver the therapeutic agents to the site of the disease. Because it is highly localized, effective concentrations of the treatment can be precisely manipulated.

The compositions described herein are particularly useful for the treatment of cancer-induced bone defects and osteoporosis. These treatments generally include delivery of the bone filler composition having an alkaline earth phosphate-based cement, nanotubes, anti-osteoclast drugs, and stem cell-derived osteoblasts, which may be delivered alone or in combination with bone augmentation surgery. In certain embodiments, the composition provides protection of bone tissue from multiple myeloma-induced
damage through a synergistic effect orchestrated by the particular combination of components as a whole. Each component by itself is useful in bone repair, but the combination of these interdisciplinary materials and methods provides various benefits over the individual components.

In the case of multiple myeloma-induced bone destruction, imbalanced regulation of the bone remodeling processes mediated by osteoblasts and osteoclasts is the underlying mechanism. Myeloma cells localize to bone marrow via interaction with bone marrow stromal cells. This association activates nuclear factor (NF)-kB dependent gene expression in bone marrow stromal cells, which leads to the secretion of cytokines, including tumor necrosis factor (TNF) alpha and interleukin-6, as well as adhesion molecules that induce myeloma cell proliferation and inhibit apoptosis through several signaling cascades. One of the major mechanisms responsible for induction of osteoclastic resorption involves association of osteoclast-expressed receptor activator of NF-kB (RANK) with its cognate ligand RANKL, a bone marrow stromal cell-secreted cytokine, which induces osteoclast differentiation from osteoclast progenitors. TNFalpha may also promote not only myeloma cell survival and proliferation, but also play
a role in osteoclast differentiation through a mechanism independent of RANKL/RANK. Therefore, medications and supplements which have been reported to inhibit osteocalst differentiation/activation, to promote osteoblast differentiation/activation, and/or to suppress tumor progression, can be used in conjunction with the cement/nanotube composition to treat cancer-induced bone diseases. These include, but are not limited to, antiresorptive drugs and anabolic drugs, anticancer agents for multiple myeloma or other tumors such as proteasome inhibitors targeting NF-kB, osteoclast-promoting cytokine antagonists, or phytochemicals and vitamins which have been shown to improved bone health.

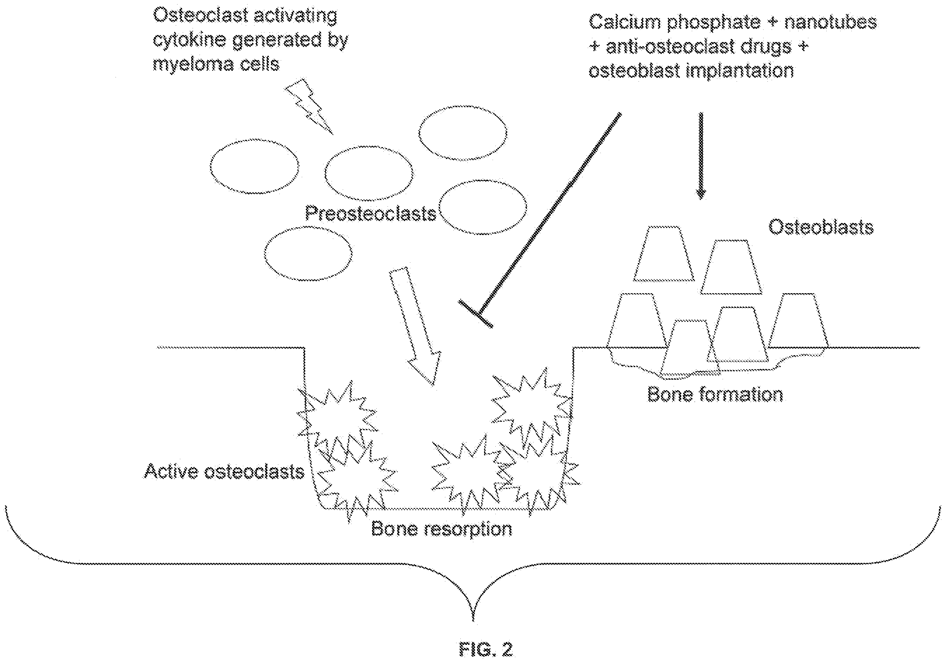

The load bearing/drug delivery system of the present disclosure can achieve localized administration of therapeutic agents to the site of bone damage to avoid unexpected side effects on other tissues. As illustrated in FIG. 2, a drug-loaded, osteoblast-incorporated calcium phosphate-based composition as described herein can inhibit bone resorption and restore bone formation. Though osteoporosis and multiple myeloma -are described herein for exemplary purposes, it is to be understood that the compositions and methods described herein can be utilized for the treatment of a wide variety of diseases or disorders. The compositions described herein can be combined with any non-invasive bone augmentation procedure, such as, but not limited to, a vertebroplasty or kyphoplasty.

Embodiments of the present disclosure (not falling under the scope of the claims) further include methods of determining coverage or denial of health insurance reimbursement and/or payment for treatments of disease or injury comprising the compositions and methods described herein. In certain embodiments (not falling under the scope of the claims), the treatment comprises the use of a bone filler material comprising an alkaline earth phosphate-based cement, nanotubes, a therapeutic agent, and osteoblasts, and a provider of health insurance denies coverage or reimbursement for the treatment.

### KITS

It is further envisioned that the compositions described herein could be embodied as parts of a kit or kits. cations. The kits may further include instructions for using the components of the kit to practice the disclosed methods, wherein the disclosed methods do not fall under the scope of the invention. The instructions for practicing the disclosed methods are generally recorded on a suitable recording medium. For example, the instructions may be present in the kits as a package insert or in the labeling of the container of the kit or components thereof. Alternatively, the instructions are present as an electronic storage data file present on a suitable computer readable storage medium, such as a flash drive, CD-ROM, or diskette. Alternatively, the actual instructions are not present in the kit, but means for obtaining the instructions from a remote source, such as via the internet, are provided. An example of this embodiment is a kit that includes a web address where the instructions can be viewed and/or from which the instructions can be downloaded. As with the instructions, this means for obtaining
the instructions is recorded on a suitable substrate.

### EXAMPLES

### Preparation of CPC Composition with Carbon Nanotubes

Cement pastes were prepared by manually mixing Ca(OH)2 with setting solution and DI water in an agate mortar by using an agate pestle. For preparing 15 mL of the setting solution, 0.0032 g of citric acid monohydrate (C₆H₈O₇·H₂O, 100%), 6 g of sodium bicarbonate (NaHCO₃, > 99.7%), 1.95 mL of DI water for diluting the setting solution, and 13.05 mL of phosphoric acid (H₃PO₄, 85%) solution as a source of phosphate, were mixed respectively. Initially, 0.6175 g of Ca(OH)₂ was mixed at least two minutes with 0.8 mL of DI water to form a paste with Ca(OH)₂ uniformly dispersed in the water. Finally, 0.75 mL of setting solution was added to the materials. The mixed paste was transferred to a household microwave with 1300 W energy input and baked for 5 minutes at maximum power. The resulting mass was crushed into fine powders using a pestle. 1 g of this monetite powder was then mixed with 0.5 mL of water or nanosilica solution to prepare a cement paste.

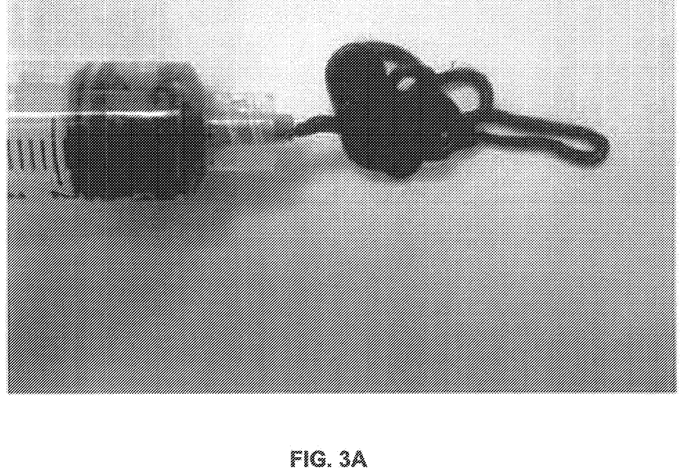

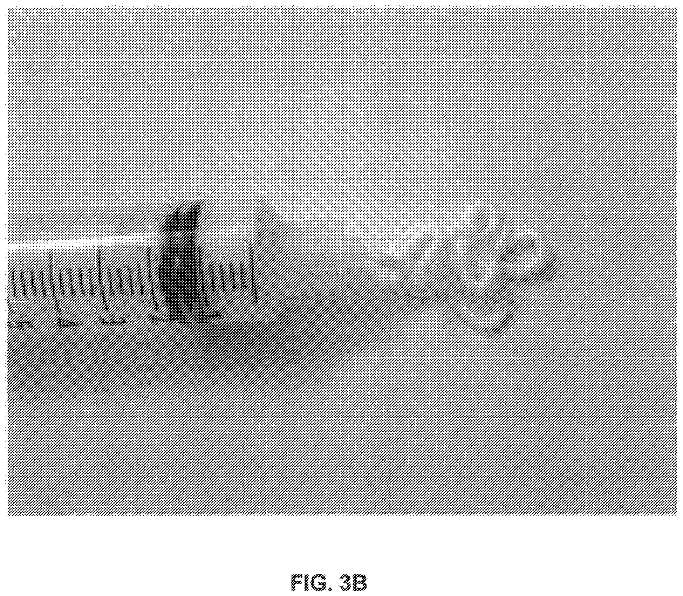

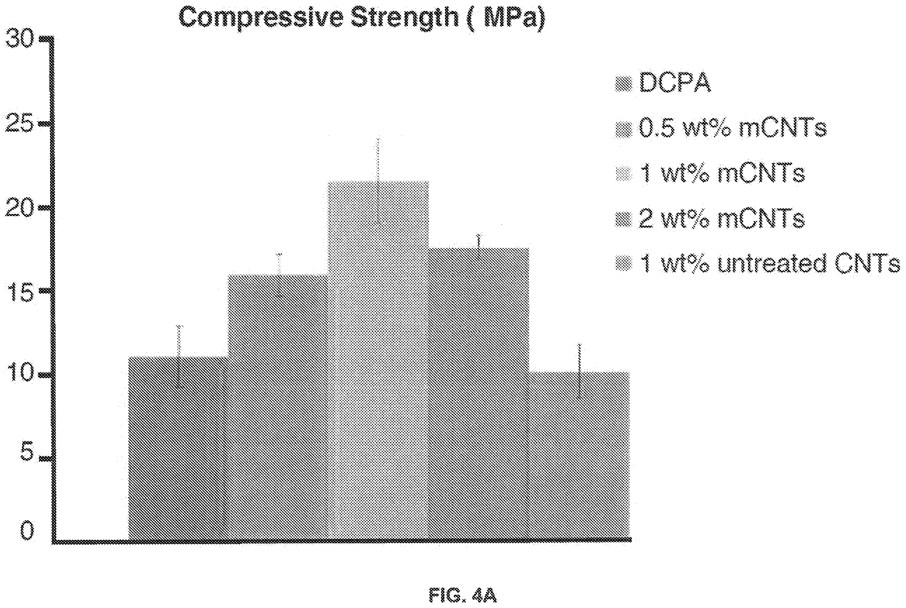

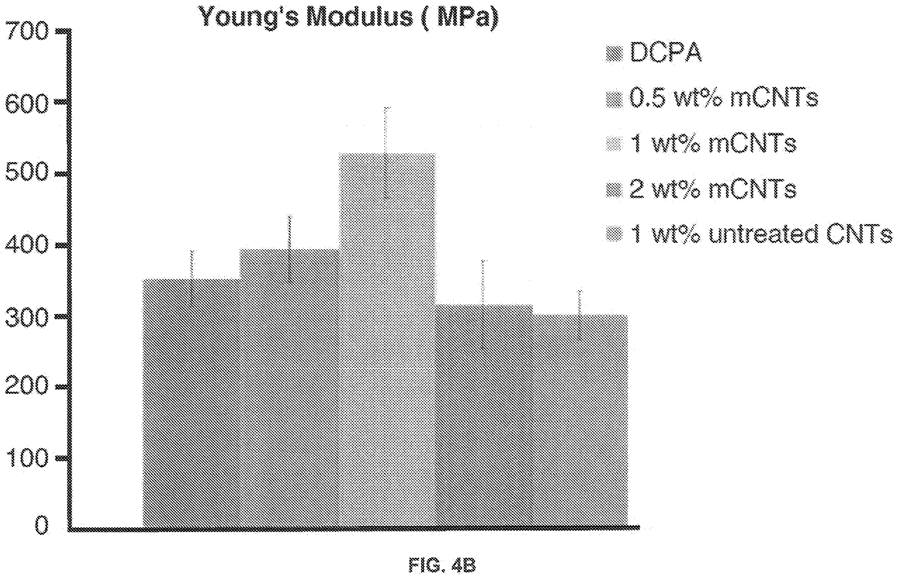

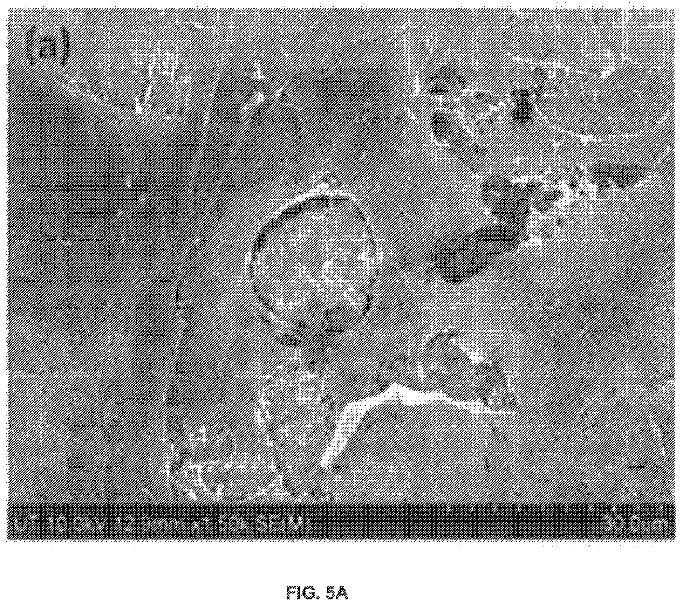

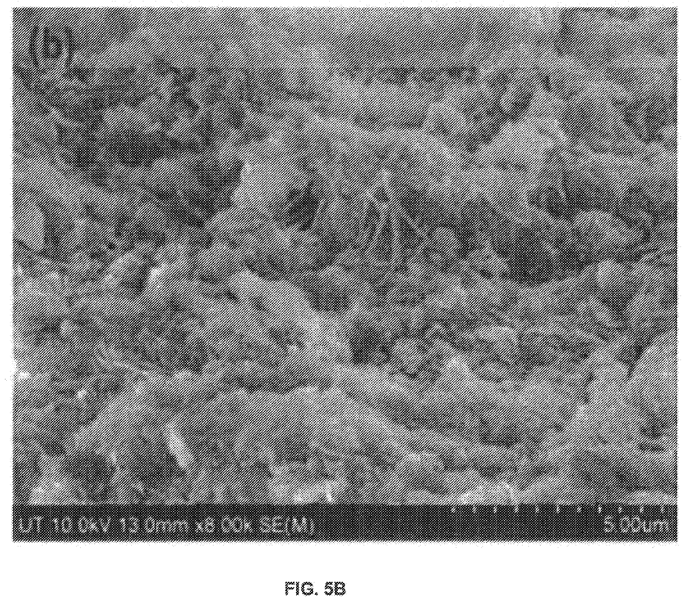

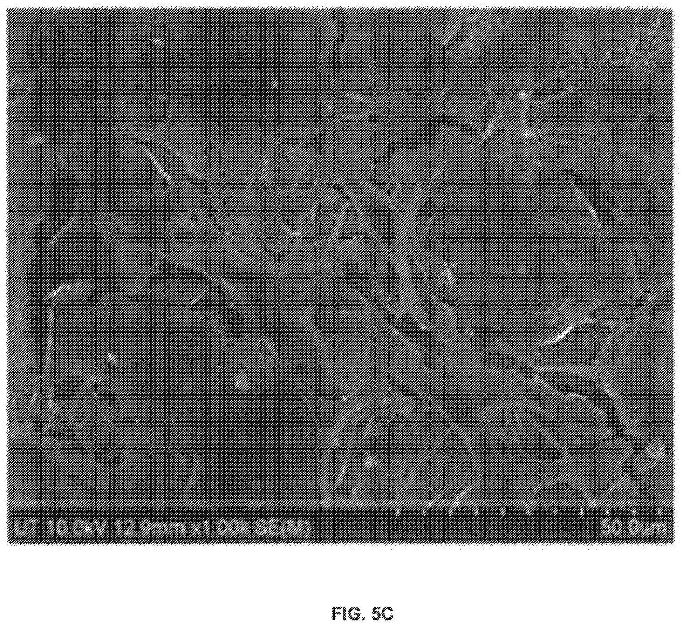

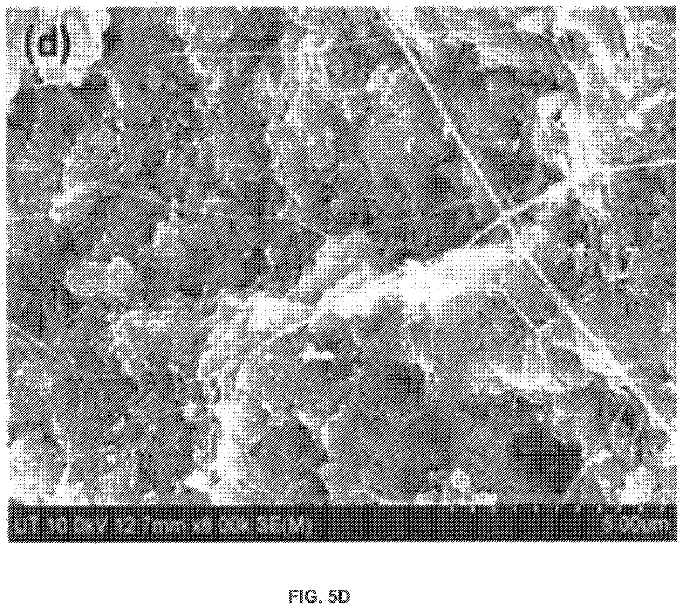

0.01 g carboxylic acid-functionalized MWCNTs was mixed with 1 g of the monetite powder while preparing the cement paste. Calcium phosphate cement with nanotubes (**FIG. 3A**) or cement alone (FIG. 3B) was extruded from a syringe with output diameter of 1.36 mm. The effect on cement setting time of varying concentrations and types of nanotubes was evaluated. Table 1 displays the observed change in setting time as a function of wt% of the carbon nanotubes. As seen from Table 1, increasing the concentration of nanotubes in the paste accelerated both the initial and final setting times. **FIGS. 3A-****3B** show that the injectability of cement paste was retained when incorporated with CNT. Handling properties (such as setting time; Table 1 and **FIG. 3**), mechanical strength (**FIG. 4**), and biocompatibility (FIG. 5) were evaluated, and the results indicate that this nanotube-reinforced cement composite is a suitable material for a bone scaffold.

**Table 1 - Cement Setting Times with Varying CNT Concentrations and Types (where mCNTs are multi-walled carboxyl-functionalized carbon nanotubes, and untreated refers to multi-walled carbon nanotubes without a carboxyl group functionalization)**

| **CNT Types** | **Percent of CNT (wt%)** | **Initial Setting Time (min)** | **Final Setting Time** (**min**) |
|---|---|---|---|
| N/A | 0 | 8 ± 2 | 36 ± 4 |
| mCNTs | 0.5 | 7 ± 3 | 31 ± 4 |
| mCNTs | 1 | 6 ± 3 | 25 ± 15 |
| mCNTs | 2 | 4 ± 2 | 26 ± 5 |
| untreated | 1 | 9 ± 3 | 39 ± 4 |

The incorporation of carbon nanotubes into the monetite (DCPA) cement was shown to improve the mechanical properties of the cement. Uniaxial compressive tests were conducted using a universal testing machine with a 50 kN load cell and a crosshead rate of loading set to 0.5 mm/min. As seen in **FIGS. 4A-4B****,** the Young's modulus, which was generated from the stress-strain plots, shows the optimal mechanical strength at cement with 1 wt% nanotubes. The compositions having 0.5 wt%, 1.0 wt%, and 2.0 wt% multi-walled carboxyl-functionalized carbon nanotubes (mCNTs) each had a greater compressive strength (MPa) than the DCPA cement alone. (**FIG. 4A****.**) The composition having untreated carbon nanotubes (that is, multi-walled carbon nanotubes without a carboxyl group functionalization) had the smallest compressive strength. Similarly, the compositions having 0.5 wt% and 1.0 wt% mCNTs both had a Young's modulus greater than that of the DCPA cement alone. (**FIG. 4B****.**)

To evaluate biocompability, osteoblasts were cultured for seven days on the calcium phosphate cement alone and on the calcium phosphate cement with multi-walled carbon nanotubes. **FIGS. 5A-5D** show SEM images of these scaffolds after seven days, which indicate the maturation of bone forming osteoblasts.

### Incmporation of WP9QY as a Therapeutic Agent

The MWCNT/CPC or CPC alone were loaded with WP9QY (YCWSQYLCY, [**SEQ ID NO: 1**]), which is a peptide compound designed to compete ligand binding of RANK and to inhibit osteoclastogensis. The WP9QY peptide has a sequence of H - Tyr - Cys - Trp - Ser - Gin - Tyr - Leu - Cys- Tyr- OH [**SEQ ID NO: 1**], and was developed based on a structural analysis of the ligand binding site on TNF receptor 1 and RANK to inhibit osteoclast-activating. This cyclic peptide mimics the most critical tumor necrosis factor (TNF) recognition loop on TNF receptor 1, and prevents interaction of TNF with its receptor. The peptide was added directly to the cement paste to form the bone filler composition.
Briefly, 100 µL of 1 mg/mL WP9QY was added into 0.5 mL DI water when mixing with 1 g of cement powder.

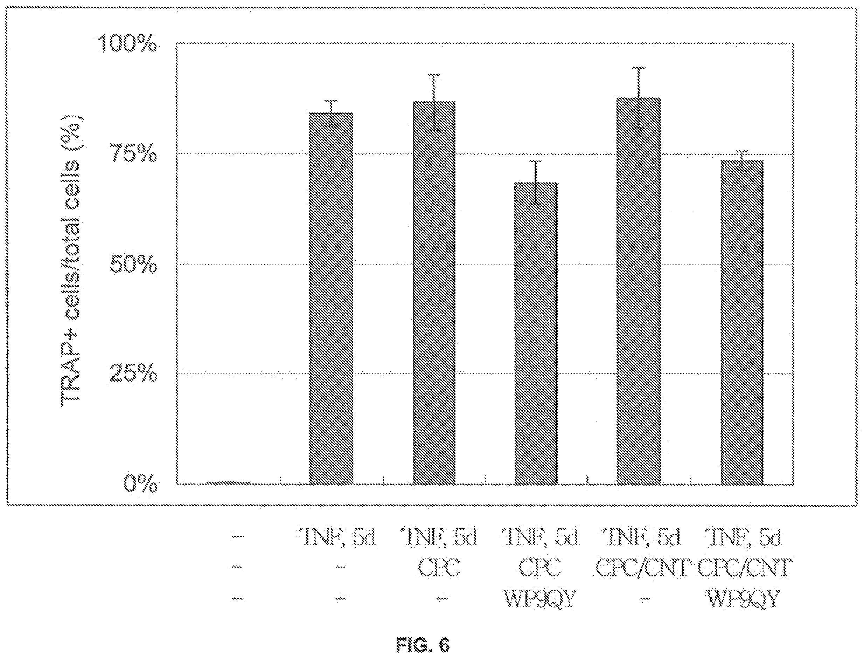

The cement pellet was washed with ethanol and left in a pre-osteoclast RAW 264.7 cell culture treated to trigger cell differentiation. To measure anti-osteoclast maturation of WP9QY released from the cement, the culture was stained for TRAP+ (osteoclast-like) cells after 5 days. TRAP+ cells are a marker for active osteoclasts after stimulated by the cytokine TNFalpha. The results, shown in **FIG. 6****,** indicate that both composites can carry and release WP9QY. As seen in **FIG. 6****,** osteoclast activation was inhibited, demonstrating the ability of the cement composition to release the peptide.

### Incorporation of MG132 as a Therapeutic Agent

A similar approach was used to investigate the drug MG132, which exhibits an inhibitory effect against NF-kB activation. MG132 is a proteasome inhibitor used to interrupt degradation of the inhibitor of NF-kB to prevent NF-kB activation and its activity of inducing gene expression responsible for osteoclastogenesis and osteolytic activity. 50 µL of 10 mM MG132 in dimethyl sulfoxide (DMSO) or 50 µL DMSO alone was added into the DI water when preparing the cement paste. The paste was left to cure in a 6-well plate at 37 °C followed by an ethanol wash and UV sterilization. Each pellet of CPC/DMSO, MG132-loaded CPC (CPC/MG132), CNT-reinforced CPC (CPC/MWCNT/DMSO), and MG132-loaded CNT -reinforced CPC (CPC/MWCNT/MG132) was immersed in 2 mL medium used to culture indicator cells, and this conditioned medium was collected at different time points. Live cell staining showed that CPC/MG132 was toxic, indicating the burst release of this cement composite. However, by adding MWCNT, this effect was attenuated, as seen in FIG. 6.

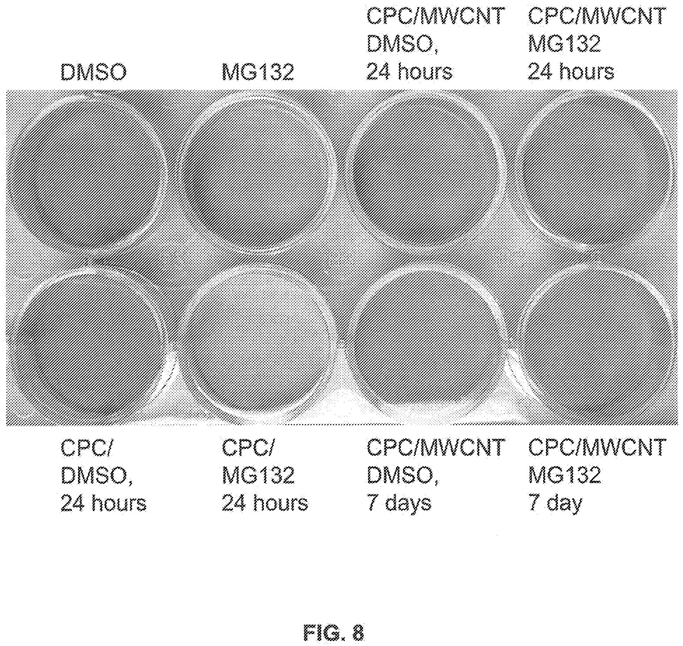

To further evaluate the release profile of the cement/drug complex, luciferase assays were performed to measure inhibition of NF-kB activation by MG132. The reporter plasmid 3XkB-Luc containing 3 copies of the NF-kB binding site was transfected in HEK293 cells. After 24 hours, culture medium was substituted by the cement/drug conditioned medium described above, collected at different time points. Cell culture was further incubated for another hour followed by an 8-hour treatment of TNFalpha (20 ng/mL). Cell lystates were prepared for luciferase activity. The results, shown in FIG. 8, indicate a more sustained release ofMG132, represented by its anti-NF-kB activity, in the nanotube-reinforced composite.

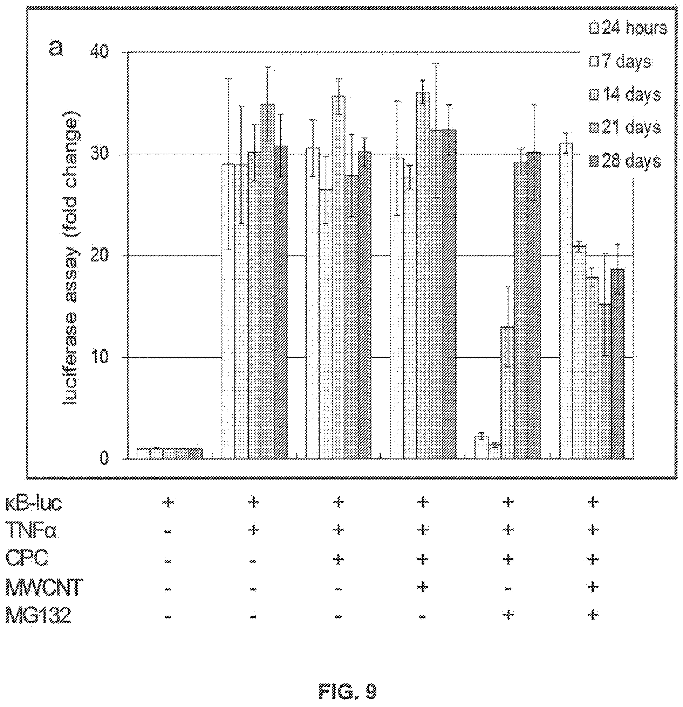

The anti-osteoclastogenesis of MG132-loaded cement was tested after a 5-day treatment of TNFalpha in RAW 264.7 culture. In this case, expression of the marker gene cathespin K was measured by real-time PCR. The results, shown in **FIG. 9**, show that osteoclast maturation can be inhibited by the MG132 released from the cement.

### Cell Encapsulation

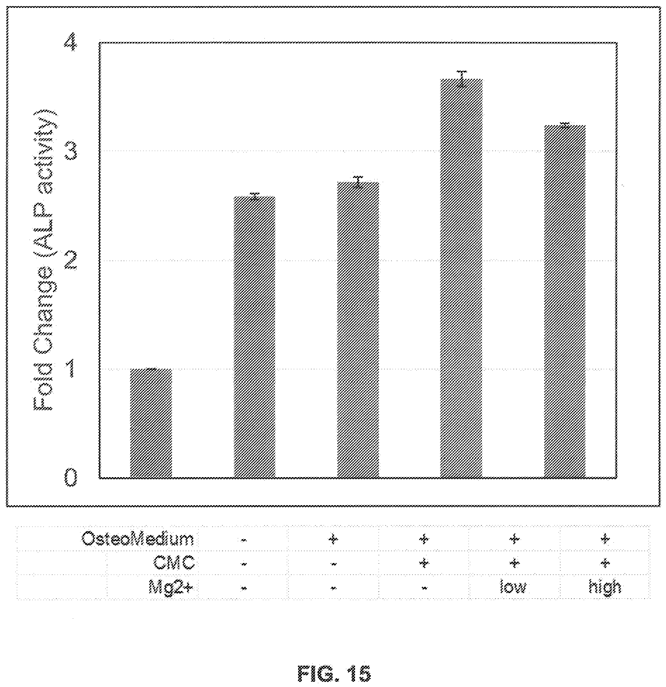

To encapsulate stem cells or osteoblasts in alginate beads, the cell suspension was prepared in 2% sodium alginic acid salt solution. Alginate/cell beads were made by adding the suspension dropwise into 2% calcium chloride using a 10 mL syringe. Micro-scale alginate beads can be produced using a coaxial air stream to drive alginate/cell droplets from a needle tip into the gelling bath. Cell suspension in 2% sodium alginic acid salt solution loaded into a syringe is fed into a bead-generating device that utilizes nitrogen gas at 10 psi to form a coaxial air flow to produce alginate droplets in 2% calcium chloride. To accelerate cell differentiation, carboxyl methyl cellulose pre-loaded with magnesium ions was added during the gelation of alginate/cell beads. After 7 days of incubation in osteogenic medium to induce osteoblast differentiation, ALP activity assays were performed to indicate the maturation of bone forming osteoblasts. The results, shown in **FIG. 15**, indicate that this alginate-based matrix provides a suitable environment for cell differentiation.

### Preparation, and Incorporation into the Cement Composition, of Peptide Nanotubes

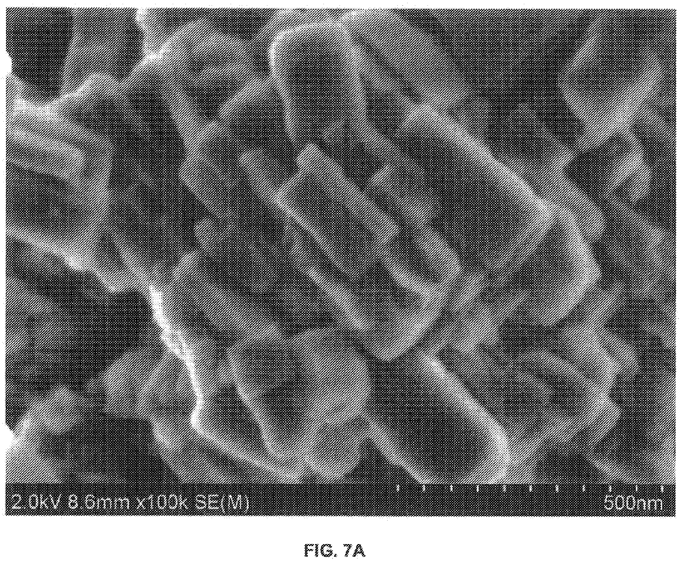

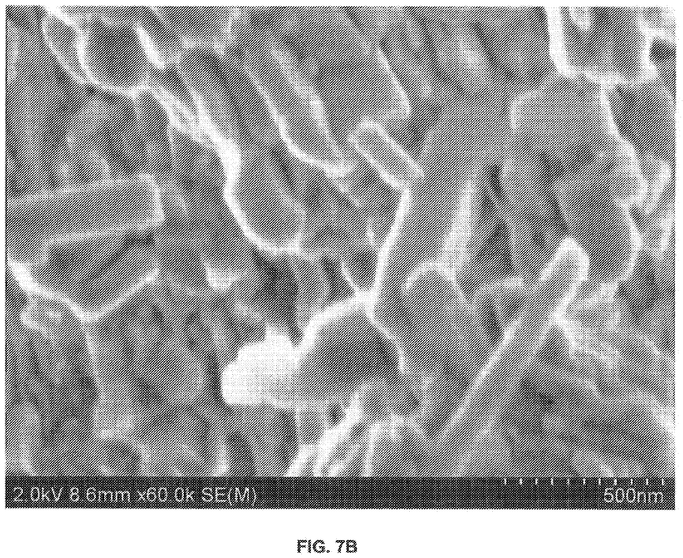

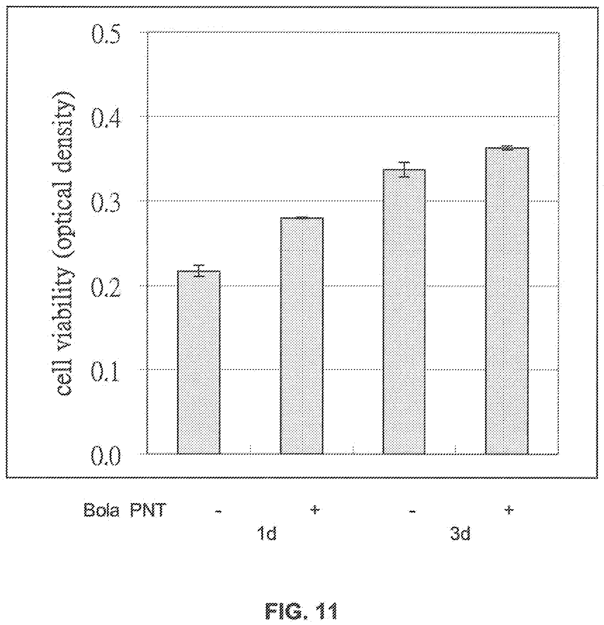

Surfactant-like Glyₙ-Aspₙ peptide nanotubes were prepared by self-assembly. The Gly₆-Asp₂ peptide [**SEQ ID NO: 2**] was outsourced for custom synthesis. To prepare the nanotubes, Gly₆-Asp₂ **[SEQ ID NO: 2]** was re-suspended in DI water, the pH was adjusted to neutral, and the suspension was sonicated to obtain a clear solution. The peptide formed nanotubular structures, which were visualized by SEM and are shown in **FIG. 7**. The result of MTT assays used to measure cell viability when the stem cells were incubated with nanotubes formed from Gly₆-Asp₂ **[SEQ ID NO: 2]** for 1 and 3 days, shown in **FIG. 11**, indicates this material is safe to use in tissue.

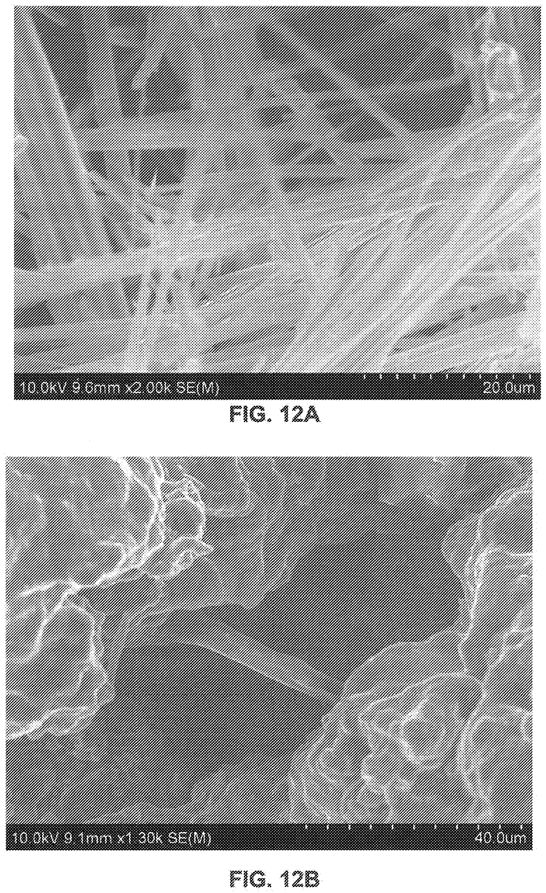

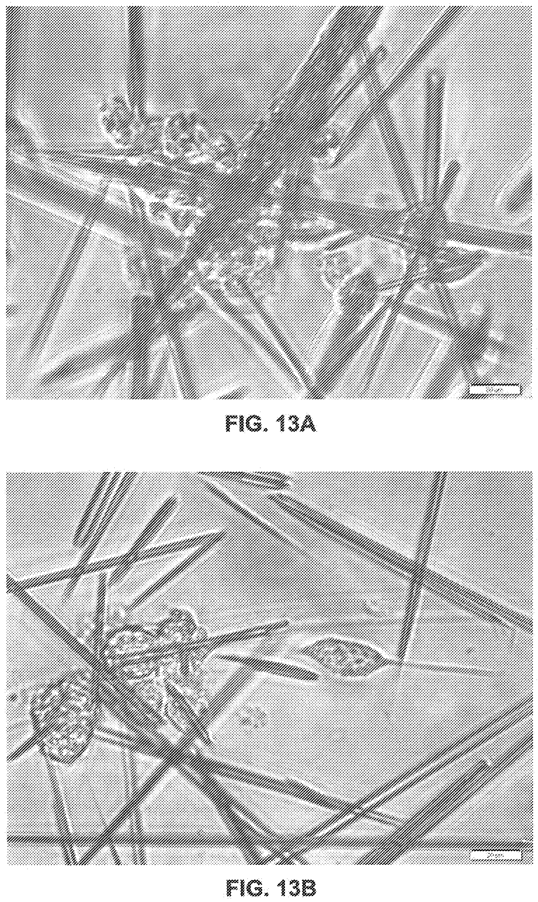

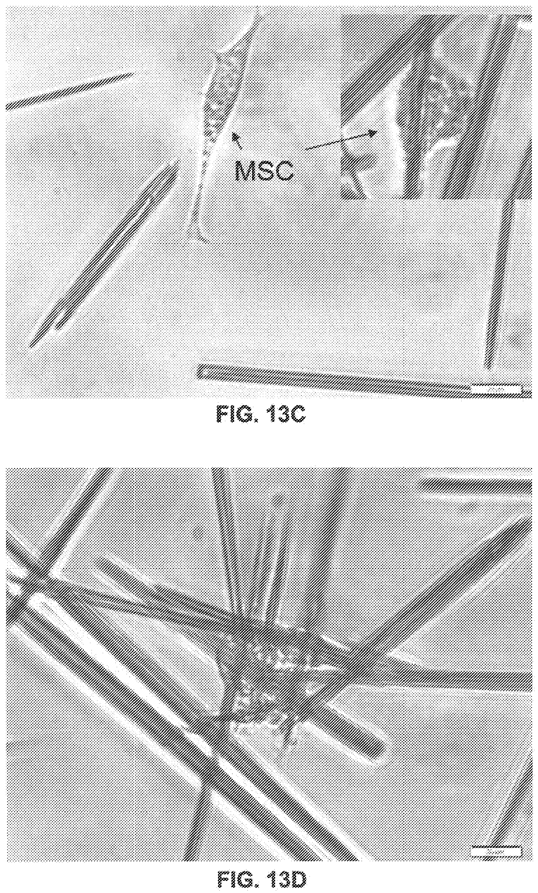

Nanotubes were also prepared from the dipeptide diphenylalanine, a modified amino acid that can form nanotubes rapidly in aqueous solution. Briefly, 100 mg/mL diphenylalanine stock solution was prepared by dissolving this molecule in 1,1,1,3,3,3-hexafluoro-2-propanol. 20 of the stock solution was added into 1 mL DI water to generate nanotubes (**FIG. 12A**). 10 mg peptide nanotubes was mixed with 1 g calcium phosphate cement during the 'Setting **(****FIG. 12B****),** showing no disruption of clystallization. These nanotubes were added to a stem cell culture to test their biocompatibility. As seen in **FIG. 12B**, the cells attached and proliferated on the nanotubes. Cell viability assays also demonstrated that cell attachment was improved on the nano-incorporated cement (**FIGS. 13A-D**).

A bolaamphiphile peptide having the assembled structure of N,N'-bis(glycylglycylglycine)hexane-1,6-dicarboxyamide can be prepared by the three-step procedure of (1) preparing glycylglycine benzyl ester hydrochloride, (2) preparing N,N'-bis(glycylglycylglycine)hexane-1,6-dicarboxyamide, and (3) preparing peptide nanotubes. The glycylglycine benzyl ester hydrochloride can be prepared as follows: both 14.8 g oft-butyloxycarbonylglycine-dicyclohexylamine and 14.0 g of glycine benzyl ester p-toluenesulfonic acid salt are added to 80 mL of chloroform. To this solution, 70 mL of chloroform solution containing 8.75 g of 1-ethyl-3-(3-dimethylaminopropyl) carboniimide hydrochloride (EDAC) is added at -5°C with stirring for 24 hours. The resulting solution is washed twice with a 10 wt% aqueous citric acid solution, then twice with water, then twice with a 4 wt% aqueous sodium hydrogen carbonate solution, and finally twice with water. The organic phase is dried and the t- butyloxycarbonyl-glycylglycine benzyl ester is obtained as a white solid. The solid is dissolved in HCl/ethylacetate and stirred for 4 hours, and then the solvent is removed *in vacuo* to obtain white precipitates. The precipitates are washed well with diethyl ether to obtain glycylgycine benzyl ester hydrochloride.

The N,N'-bis(glycylglycylglycine)hexane-1,6-dicarboxyamide can be prepared as follows: both 1,6-hexanedicarboxylic acid (0.50 g) and 1-hydroxylbenzotriazole (0.65 g) are dissolved in 10 mL of N,N-dimethylformamide (DMF'). To this solution, 10 mL of a chloroform solution containing 0.915 g of
EDAC is added at -5°C with stirring for one hour. To the resulting solution, 10 mL of a methanol solution containing 1.24 g of glycylglycine benzyl ester hydrochloride prepared in step (1), and then 0.67 mL of triethylamine are successively added. The mixture is stirred for 24 hours while gradually increasing the temperature to room temperature. Then, the solvent is removed *in vacuo* to obtain the precipitates. The preciphates are then washed on a filter paper with 10 wt% citric acid solution, 20 mL of water, and 50 mol of 4 wt% aqueous sodium hydrogen carbonate solution, and then crystallized from DMF to obtain N,N-bis(glycylglycine benzyl ester)-hexane-1,6-dicarboxyamide. This dicarboxyamide is dissolved in DMF in a water bath at 50 °C, to which 0.25 g of a catalyst (10 wt% palladium on carbon) is added for a 6-hour reaction. After the reaction, the catalyst is removed by filtration, and then the filtrate
is recrystalized from DMF to obtain N,N'-bis(glycylglycine)-hexane-1,6-dicarboxyamide.

Step (3), preparation of the oligopeptide heptanes bolaamphiphile nanotubes, can be performed by mixing the oligopeptide bolaamphiphile with 10 mL of distilled water containing 8 mg (0.2 mmol) of sodium hydroxide, and then sonicating the mixture to obtain a solution. This solution is allowed to quiescently stand at room temperature in air for 2 weeks. The supernatant is removed by decantation and the precipitates are dehydrated and completely dried in a high vacuum to obtain fibrous microtubes.

### Preparation of Magnesium Phosphate Cement

Magnesium phosphate cement was prepared by manually mixing Mg(OH)₂ with setting solution and DI water in an agate mortar by using an agate pestle. For preparing 15 mL of the setting solution, 0.0032 g of citric acid monohydrate (C₆H₈O₇·H₂O, 100%), 6 g of sodium bicarbonate (NaHC03, >99.7%), 1.95 mL ofDI water for diluting the setting solution, and 13.05 mL of phosphoric acid (H3P04, 85%) solution as a source of phosphate, were mixed respectively. 2.47 g of Mg(OH)₂ was added to a mixture of 2 mL of DI water and 3 mL of setting solution to form a paste. The mixed paste was transferred to a household microwave with 1300 W energy input and baked for 5 minutes at maximum power. The resulting mass was crushed into fine powder using a pestle. 2 g of the MPC powder was then mixed with 0.5 mL water or nanosilica solution to generate an injectable magnesium phosphate cement.

### Integration of Cement, Nanotubular Structures, Therapeutic Agents, and Cells

Integration of the cement, nanotubes, therapeutic agents, and alginate-encapsulated cells described in these examples can be achieved by homogeneously mixing 1g CPC or 2 g MPC powder with 1% w/w carbon nanotubes or dried peptide nanotubes in 0.5 mL DI water or nanosilica solution with pre- dissolved 50µL of 10 mM MG132 or 100 µl of 1 mg/mL WP9QY. Alginate/carboxyl methyl cellulose/osteoblast or alginate/carboxyl cellulose/stem cell beads can then be added to this paste, and the resulting complex is ready for use in bone augmentation.

## Claims

1. A bone filler composition comprising:
an alkaline earth phosphate-based cement consisting essentially of monetite;
nanotubular structures comprising carboxyl-functionalized multi-walled carbon nanotubes; and
at least one of: a therapeutic agent or cells;
wherein the composition is a paste that sets into a hardened mass, and the hardened mass is capable of releasing the therapeutic agent or cells.

2. The composition of claim 1, wherein the nanotubular structures further comprise peptide nanotubes.

3. The composition of claim 2, wherein the peptide nanotubes are formed from Glyₙ-Aspₙ peptides, wherein each n is independently from 1 to 10 **[SEQ ID NO: 3].**

4. The composition of claim 2, wherein the peptide nanotubes comprise the peptide Gly-Gly-Gly-Gly-Gly-Gly-Asp-Asp **[SEQ ID NO: 2].**

5. The composition of claim 2, wherein the peptide nanotubes comprise a compound selected from the group consisting of: N,N'-bis(glycyl-glycine)-hexane-1,6-dicarboxyamide; N,N'-Bis (glycylglycylglycine)octadecane-1,18-dicarboxyamide; and N,N'-bis(glycylglycine)decane-1,10-dicarboxyamide.

6. The composition of claim 2, wherein the peptide nanotubes comprise ionic complementary peptides.

7. The composition of claim 1, wherein the therapeutic agent comprises an antagonist of a signal transduction responsible for osteoclast differentiation or activation.

8. The composition of claim 1, wherein the therapeutic agent comprises a peptide having the sequence YCWSQYLCY [**SEQ ID NO: 1**].

9. The composition of claim 1, wherein the cells comprise mesenchymal stem cells or osteoblasts derived from mesenchymal stem cells.

10. The composition of claim 9, wherein the osteoblasts are encapsulated in alginate beads comprising carboxy methyl cellulose.

11. A method of preparing a bone filler material complex comprising:
preparing an alkaline earth phosphate-based bone cement powder consisting essentially of monetite;
mixing the monetite powder with nanotubes and pre-dissolved therapeutic agents to form a paste, wherein the nanotubes comprise carboxyl-functionalized multi-walled carbon nanotubes; and
adding encapsulated cells to the paste to form a complex.

12. The method of claim 11, wherein the cells are osteoblasts derived from mesenchymal stem cells and wherein the osteoblasts are encapsulated in alginate beads.

## Patentansprüche

1. Knochenfüllstoffzusammensetzung, umfassend:
einen Zement auf Erdalkaliphosphatbasis, der im Wesentlichen aus Monetit besteht;
nanoröhrenförmige Strukturen, umfassend mit Carboxyl funktionalisierte, mehrwandige Kohlenstoff-Nanoröhren; und
mindestens eines aus: einem therapeutischen Wirkstoff oder Zellen;
wobei die Zusammensetzung eine Paste ist, die zu einer gehärteten Masse aushärtet, und die gehärtete Masse befähigt ist, den therapeutischen Wirkstoff oder die Zellen freizusetzen.

2. Zusammensetzung nach Anspruch 1, wobei die nanoröhrenförmigen Strukturen weiter Peptid-Nanoröhren umfassen.

3. Zusammensetzung nach Anspruch 2, wobei die Peptid-Nanoröhren aus Glyₙ-Aspₙ Peptiden gebildet sind, wobei jedes n unabhängig aus 1 bis 10 ist **[SEQ ID NO: 3].**

4. Zusammensetzung nach Anspruch 2, wobei die Peptid-Nanoröhren das Peptid Gly-Gly-Gly-Gly-Gly-Gly-Asp-Asp **[SEQ ID NO: 2]** umfassen.

5. Zusammensetzung nach Anspruch 2, wobei die Peptid-Nanoröhren eine Verbindung umfassen, ausgewählt aus der Gruppe, bestehend aus:
N,N'-Bis(glycylglycin)-hexan-1,6-dicarboxyamid;
N,N'-Bis(glycylglycylglycin)octadecan-1,18-dicarboxyamid; und
N,N'-Bis(glycylglycin)decan-1,10-dicarboxyamid.

6. Zusammensetzung nach Anspruch 2, wobei die Peptid-Nanoröhren ionisch komplementäre Peptide umfassen.

7. Zusammensetzung nach Anspruch 1, wobei der therapeutische Wirkstoff einen Antagonisten einer Signaltransduktion, die für die Differenzierung oder Aktivierung von Osteoklasten verantwortlich ist, umfasst.

8. Zusammensetzung nach Anspruch 1, wobei der therapeutische Wirkstoff ein Peptid mit der Sequenz YCWSQYLCY **[SEQ ID NO: 1]** umfasst.

9. Zusammensetzung nach Anspruch 1, wobei die Zellen mesenchymale Stammzellen oder von mesenychmalen Stammzellen abgeleitete Osteoblasten umfassen.

10. Zusammensetzung nach Anspruch 9, wobei die Osteoblasten in Alginatbeads verkapselt sind, die Carboxymethylcellulose umfassen.

11. Verfahren zur Herstellung eines Knochenfüllstoffmaterialkomplexes, umfassend:
Herstellen eines Knochenzementpulvers auf Erdalkaliphosphatbasis, das im Wesentlichen aus Monetit besteht;
Mischen des Monetit-Pulvers mit Nanoröhren und vorab aufgelösten therapeutischen Wirkstoffen, um eine Paste zu bilden, wobei die Nanoröhren mit Carboxyl funktionalisierte, mehrwandige Kohlenstoff-Nanoröhren umfassen; und
Hinzufügen verkapselter Zellen zu der Paste, um einen Komplex zu bilden.

12. Verfahren nach Anspruch 11, wobei die Zellen von mesenychmalen Stammzellen abgeleitete Osteoblasten sind, und wobei die Osteoblasten in Alginatbeads verkapselt sind.

## Revendications

1. Composition de matière de remplissage osseux comprenant :
un ciment à base de phosphate alcalino-terreux essentiellement constitué de monétite ;
des structures nanotubulaires comprenant des nanotubes de carbone à parois multiples à fonction carboxyle ; et
au moins l'un parmi : un agent thérapeutique ou des cellules ;
où ladite composition est une pâte qui se solidifie sous forme d'une masse durcie, et la masse durcie est capable de libérer l'agent thérapeutique ou les cellules.

2. Composition selon la revendication 1, dans laquelle les structures nanotubulaires comprennent en outre des nanotubes de peptides.

3. Composition selon la revendication 2, dans laquelle les nanotubes de peptides sont formés à partir de peptides Glyₙ-Aspₙ, dans lesquels chaque n varie indépendamment de 1 à 10 [**SEQ ID NO : 3**]**.**

4. Composition selon la revendication 2, dans laquelle les nanotubes de peptides comprennent le peptide Gly-Gly-Gly-Gly-Gly-Gly-Asp-Asp [**SEQ ID NO : 2**].

5. Composition selon la revendication 2, dans laquelle les nanotubes de peptides comprennent un composé choisi dans le groupe constitué par: le N,N'-bis(glycyl-glycine)-hexane-1,6-dicarboxyamide ; le N,N'-bis(glycylglycylglycine)octadécane-1,18-dicarboxyamide ; et le N,N'-bis(glycylglycine)décane-1,10-dicarboxyamide.

6. Composition selon la revendication 2, dans laquelle les nanotubes de peptides comprennent des peptides ioniques complémentaires.

7. Composition selon la revendication 1, dans laquelle l'agent thérapeutique comprend un antagoniste d'une transduction de signal responsable de la différenciation ou de l'activation des ostéoclastes.

8. Composition selon la revendication 1, dans laquelle l'agent thérapeutique comprend un peptide ayant la séquence YCWSQYLCY [**SEQ ID NO : 1**].

9. Composition selon la revendication 1, dans laquelle les cellules comprennent les cellules souches mésenchymateuses ou les ostéoblastes dérivés de cellules souches mésenchymateuses.

10. Composition selon la revendication 9, dans laquelle les ostéoblastes sont encapsulés dans des billes d'alginate comprenant de la carboxyméthylcellulose.

11. Procédé de préparation d'un complexe de matière de remplissage osseux comprenant :
la préparation d'une poudre de ciment osseux à base de phosphate alcalino-terreux constituée essentiellement de monétite ;
le mélange de la poudre de monétite avec des nanotubes et des agents thérapeutiques pré-dissous pour former une pâte, où les nanotubes comprennent des nanotubes de carbone à parois multiples à fonction carboxyle ; et
l'ajout de cellules encapsulées à la pâte pour former un complexe.

12. Procédé selon la revendication 11, dans lequel les cellules sont des ostéoblastes dérivés de cellules souches mésenchymateuses et dans lequel les ostéoblastes sont encapsulés dans des billes d'alginate.


### SEQUENCE LISTING

<110> THE UNIVERSITY OF TOLEDO
<120> INJECTABLE BIODEGRADABLE BONE MATRIX FOR MULTIPLE MYELOMA LESION AUGMENTATION AND OSTEOPOROSIS
<130> 53-54894/D2013-62
<140>
   <141>
<150> 61/783,100
   <151> 2013-03-14
<160> 3
<170> PatentIn version 3.5
<210> 1
   <211> 9
   <212> PRT
   <213> Artificial Sequence
<220>
   <223> Description of Artificial Sequence: Synthetic peptide
<400> 1
   Tyr Cys Trp Ser Gln Tyr Leu Cys Tyr
   1               5
<210> 2
   <211> 8
   <212> PRT
   <213> Artificial Sequence
<220>
   <223> Description of Artificial Sequence: Synthetic peptide
<400> 2
   Gly Gly Gly Gly Gly Gly Asp Asp
   1               5
<210> 3
   <211> 20
   <212> PRT
   <213> Artificial Sequence
<220>
   <223> Description of Artificial Sequence: Synthetic peptide
<220>
   <221> misc_feature
   <222> (1)..(10)
   <223> This region may encompass 1-10 'Gly' residues
<220>
   <221> misc_feature
   <222> (11)..(20)
   <223> This region may encompass 1-10 'Asp' residues
<400> 3
   Gly Gly Gly Gly Gly Gly Gly Gly Gly Gly Asp Asp Asp Asp Asp Asp
   1               5                   10                  15
   Asp Asp Asp Asp
               20